# EUROPEAN PATENT APPLICATION

(11) **EP 3 943 091 A2**
(43) Date of publication of application: **26.01.2022**
(21) Application number: 20774251.1
(22) Date of filing: 18.03.2020
(51) Int. Cl.: A61K 35/28, A61P 17/00, A61P 29/00, C12N 5/0775

(54) **METHOD FOR TREATING ATOPIC DERMATITIS USING MONOCLONAL STEM CELLS**

(30) Priority: 19.03.2019 KR 20190030900
(71) Applicant: SCM Lifescience Co., Ltd., Jung-gu Incheon 22332 (KR)
(72) Inventor: SONG, Sun Uk, Incheon 22003 (KR); CHOI, Gwang Seong, Seoul 07981 (KR); CHO, Yun Kyoung, Incheon 22187 (KR); KIM, Si Na, Incheon 21997 (KR); CHUNG, Eun Kyung, Seoul 08803 (KR)
(74) Representative: Altmann Stößel Dick Patentanwälte PartG mbB
(86) International application number: PCT/KR2020/003694
(87) International publication number: WO 2020/190023

(57) **Abstract**

The present invention pertains to a method and a composition for preventing or treating atopic dermatitis through the administration of monoclonal stem cells. The present invention also pertains to a composition and a method for screening atopic dermatitis patients suitable for the administration of monoclonal stem cells and predicting the prognosis of the patients in order to apply the prevention or treatment method and composition. According to an improved method for the subfractionation culture and proliferation of stem cells of the present invention, large quantities of desired monoclonal mesenchymal stem cells can be obtained in a short period of time through the rapid proliferation of monoclonal mesenchymal stem cells. In addition, the monoclonal mesenchymal stem cells thus obtained can be administered to atopic dermatitis patients according to a prescribed administration cycle, guidelines, and dose to effectively ameliorate atopy symptoms in the patients. Moreover, by using a marker to identify patient groups particularly suitable for the treatment and selectively administering the monoclonal mesenchymal stem cells to the patient groups, an excellent therapeutic effect for atopic dermatitis can be achieved.

## Description

### [Technical Field]

The present invention relates to a method and composition for preventing or treating atopic dermatitis by administering monoclonal stem cells. In addition, the present invention relates to a composition and method for screening atopic dermatitis patients suitable for monoclonal stem cell administration and predicting their prognosis in order to apply the method and composition.

### [Background Art]

Atopic dermatitis (AD), also known as atopic eczema, is a very common inflammatory skin disease. There are reports that the pathogenesis of acute atopic dermatitis is the infiltration of CD4+T cells and eosinophils into the skin and Th2 inflammatory response mediated by increased secretion of immunoglobulin E (IgE) and Th2 cytokines. Atopic dermatitis is accompanied by symptoms such as severe itching and dry skin. Atopic dermatitis has characteristics such as a high level of IgE expression in the blood and an increase in eosinophils. Recently, it is estimated that atopic dermatitis occurs in about 10 to 20% of the total population, but there is no clear treatment that can completely cure it. In particular, most are diagnosed at a young age, under 5 years of age, and 50% of them are diagnosed between 6 and 24 months of age. A nationwide epidemiological survey conducted by the Korean Pediatric Allergy and Respiratory Association showed that the prevalence of atopic dermatitis has been increasing for the past 10 years, and social interest in it is increasing. Since 50 to 75% of children with atopic dermatitis show the course of allergic disease that progresses to asthma and rhinitis, early diagnosis and management of atopic dermatitis, the starting point of the progression to allergy, is very important for preventing the progression of allergy in adults.

Atopic dermatitis is caused by many factors such as immunological abnormalities such as T lymphocyte activation, abnormal cytokine system, cell-mediated immunity decrease, and IgE increase, physiological factors, and biochemical defects of the skin. In order to treat such atopic dermatitis, treatment using drugs such as steroids, as well as moisturizing of dry skin, is typically required. As a treatment for atopic dermatitis, if the symptoms are mild, a moisturizer, a topical steroid, an antihistamine, an antibiotic, and a local immune response modifier are used. In case of severe atopic dermatitis, systemic steroids or immunosuppressants are used, but long-term use has side effects, and if taking the drugs is stopped, there is a high possibility of recurrence of the lesion, so a safe and effective treatment for long term use is required.

Recently, attempts have been made to use stem cells for the treatment of various inflammatory diseases. Stem cells have the potential to grow into tissues of all 210 organs in our body. The stem can divide infinitely and can differentiate into desired organs by appropriate manipulation. Due to these characteristics of stem cells, stem cells are in the spotlight as new therapeutic agents. The possibility of treating incurable diseases using stem cells is very high. Thus, it is expected that stem cells may treat numerous diseases such as leukemia, osteoporosis, hepatitis, Parkinson's disease, senile dementia, and burns.

However, stem cells still have many limitations because it is difficult to obtain them in large quantities. Although the method of obtaining stem cells from frozen embryonic cells is efficient, there are still many ethical issues. In order to solve this problem, many studies have been conducted on a method for obtaining stem cells using a somatic cell nuclear transfer method or an adult stem cell. Adult stem cell research is more active than embryonic stem cell research. Adult stem cells remain in various organs, such as the central nervous system and bone marrow, and thus participate in organ development during the growth phase and regenerationat the time of injury. Because they are present in various organs, they can be obtained from various sites including bone marrow, spleen, and fat cells, but the method obtained from bone marrow is the most commonly performed. However, it is difficult to always obtain uniform cells in the process of isolating and culturing mesenchymal stem cells among many types of bone marrow cells. Thus, studies are being conducted to supplement these problems.

The present inventors have invented a novel method for isolating stem cells called a subfractionation culturing method and applied for a patent through Korean patent application No. 10-2006-0075676, which has been issued. The subfractionation culturing method has excellent superiority compared to other methods of obtaining stem cells because it can be performed at a low cost, it does not have any problem of contamination, and it can effectively obtain clonal mesenchymal stem cells (cMSCs) without the possibility of mixing with other stem cells. However, despite the superiority of the subfractionation culturing method, in order to mass-produce mesenchymal stem cells and use them as a final product, a sufficient amount of mesenchymal stem cells can be obtained only through a process of preparing a working cell bank and obtaining a final product through it. There was a limitation in that it is difficult to obtain a rapid monoclonal mesenchymal stem cell population because the culture of at least 10 passages is required.

Further, there is a lack of research on whether stem cells can effectively alleviate various clinical symptoms of atopic dermatitis by administering stem cells to individuals in need of treatment. There is also no research on information on patient groups that can be expected to have an excellent effect by stem cell administration. Thus, it is necessary to solve the problem of mass production of stem cells that may exhibit excellent therapeutic effects for atopic dermatitis, and at the same time, establish a novel treatment method that can treat atopic dermatitis using this.

### [Disclosure]

### [Technical Problem]

The present inventors have researched an effective method for treating atopic dermatitis using stem cells and have found that when the monoclonal mesenchymal stem cells obtained by the present applicant's original method are administered to an appropriate patient group at a specific cell concentration and cycle, atopic dermatitis may be effectively treated, thereby completing the present invention.

Accordingly, an object of the present invention is to provide a composition for screening atopic dermatitis patients suitable for administration of monoclonal stem cells or composition for predicting the prognosis of atopic dermatitis patients according to the administration of monoclonal stem cells, the composition including one or more selected from the group consisting of IL-10, IL-13, IFNg, IL-5 and IL-17; or an agent for measuring their expression.

Another object of the present invention is to provide a method for screening atopic dermatitis patients suitable for a method for administration of monoclonal stem cells and a method for providing information on prognosis prediction of atopic dermatitis treatment according to the administration of monoclonal stem cells, the method including a step of measuring one or more selected from the group consisting of IL-10, IL-13, IFNg, IL-5 and IL-17 in a sample of atopic dermatitis patient.

Still another object of the present invention is to provide a pharmaceutical composition for preventing or treating atopic dermatitis, the composition including monoclonal stem cells for administration to a specific patient group.

Yet another object of the present invention is to provide a method for treating atopic dermatitis, the method including a step of administering monoclonal stem cells obtained by the method of the present invention to an individual at a dose of 1 × 10³ cells/kg to 1 × 10⁸ cells/kg at intervals of 1 week to 3 weeks.

### [Technical Solution]

In order to achieve the object, the present invention provides a composition for screening atopic dermatitis patients suitable for administration of monoclonal stem cells, the composition including one or more selected from the group consisting of IL-10, IL-13, IFNg, IL-5 and IL-17; or an agent for measuring their expression.

Further, the present invention provides a composition for predicting the prognosis of atopic dermatitis patients according to the administration of monoclonal stem cells, the composition including one or more selected from the group consisting of IL-10, IL-13, IFNg, IL-5 and IL-17; or an agent for measuring their expression.

Further, the present invention provides a kit for screening atopic dermatitis patients suitable for administration of monoclonal stem cells, the kit including the composition for the selection.

Further, the present invention provides a kit for predicting the prognosis of atopic dermatitis patients according to the administration of monoclonal stem cells, the kit including the composition for prediction of prognosis.

Further, the present invention provides a method for screening atopic dermatitis patients suitable for a method for administration of monoclonal stem cells, the method including a step of measuring the expression of one or more selected from the group consisting of IL-10, IL-13, IFNg, IL-5 and IL-17 in a sample of atopic dermatitis patient.

Further, the present invention provides a method for providing information on prognosis prediction of atopic dermatitis treatment according to the administration of monoclonal stem cells, the method including a step of measuring the expression of one or more selected from the group consisting of IL-10, IL-13, IFNg, IL-5 and IL-17 in a sample of atopic dermatitis patient.

Further, the present invention provides a pharmaceutical composition for preventing or treating atopic dermatitis including monoclonal stem cells, in which the pharmaceutical composition is for administration when before treatment, the patient's i) IL-10 is 20 pg/ml or more and IL-13 is 100 pg/ml or more, ii) IFNg and IL-5 are not detected, or iii) IL-17 is detected.

Further, the present invention provides a method for treating atopic dermatitis, the method including steps of 1) culturing the bone marrow isolated from the individual in a first vessel; 2) transferring only the supernatant from the first vessel to a new vessel and culturing the supernatant; 3) culturing the cells present in the new vessel and obtaining a supernatant; 4) repeating step 2) and 3) one or more times in which the supernatant of step 3) is used as the supernatant of the first vessel of step 2) to obtain monoclonal stem cells; 5) inoculating the monoclonal stem cells of step 4) in a medium at a cell density of 50 to 1,000 cells/cm² and culturing the cells to obtain monoclonal stem cells; and 6) administering the obtained monoclonal stem cells to an individual at a dose of 1 × 10³ to 1 × 10⁸ cells/kg at an interval of 1 week to 3 weeks.

### [Advantageous Effects]

According to the improved subfractionation culture and proliferation and proliferation method of stem cells of the present invention, it is possible to obtain a large amount of desired monoclonal mesenchymal stem cells in a short time through the rapid proliferation of monoclonal mesenchymal stem cells. Further, the monoclonal mesenchymal stem cells obtained through this method is administered to an atopic dermatitis patient at a prescribed administration cycle, usage, and dose so that it is possible not only to effectively alleviate the atopic symptoms of the patient, but also to specifically confirm a patient group suitable for this treatment according to markers and selectively administer the cells, thereby achieving an excellent therapeutic effect for atopic dermatitis.

### [Description of Drawings]

FIG. 1 shows a conventional subfractionation culturing method for isolating monoclonal mesenchymal stem cells from bone marrow.
FIG. 2 shows the results of confirming the morphological changes of monoclonal mesenchymal stem cells according to cell culture density and cell subculture through microscopic observation.
FIG. 3 shows the results of confirming the change in cell size and granularity of monoclonal mesenchymal stem cells according to cell culture density and cell subculture by mean of forward scatter (FSC) (A) and side scatter (SSC) (B) light through flow cytometry (FACS) analysis (^{∗}p < 0.05, ^{∗∗}p < 0.01, ^{∗∗∗}p < 0.005).
FIG. 4 shows the results of confirming whether cells are aged after staining monoclonal mesenchymal stem cells by beta-gal-staining in which cell culture densities and cell subcultures vary.
FIG. 5 shows the results of RT-PCR of passage 15(P15) and passage 16(P16), which are age-related genes and PCNA, which is a proliferation marker obtained after culturing the monoclonal mesenchymal stem cells of P15 while the cell culture density varies.
FIG. 6 shows the results of confirming the proliferation ability of monoclonal mesenchymal stem cells by cell population doubling time (PDT) and cell population doubling level (PDL) according to cell culture density and cell subculture. (^{∗}p < 0.05, ^{∗∗}p < 0.01, ^{∗∗∗}p < 0.005).

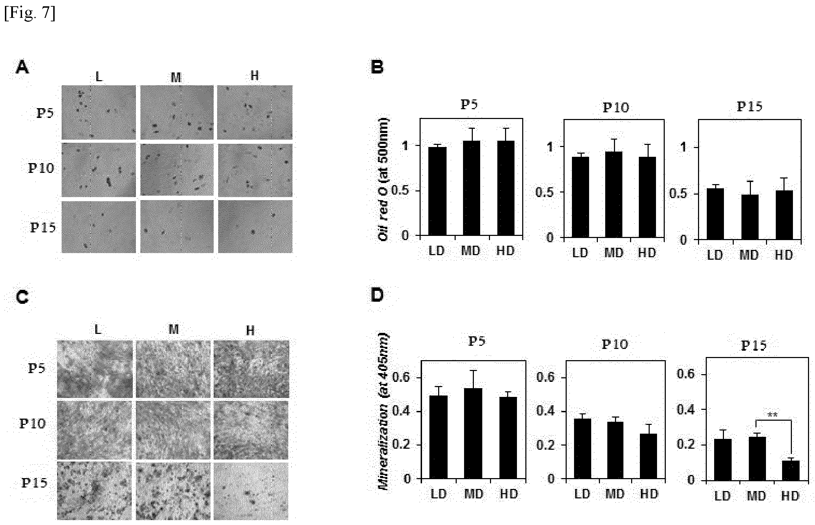

FIG. 7 shows the results of confirming the differentiation ability of monoclonal mesenchymal stem cells according to cell culture density and cell passage (^{∗}p < 0.05, ^{∗∗}p < 0.01, ^{∗∗∗}p < 0.005). FIG. 7A shows the result of confirming the differentiation ability of monoclonal mesenchymal stem cells into adipocytes according to cell culture and cell subculture through Oil red O histological staining. FIG. 7B shows the results of quantifying the level of histological staining described in FIG. 7A. FIG. 7C shows the result of confirming the differentiation ability of monoclonal mesenchymal stem cells into osteogenic cells according to cell culture and cell subculture through Alizarin red S histological staining. FIG. 7D shows the result of quantifying the level of histological staining described in FIG. 7C
FIG. 8 shows the results of confirming total reactive oxygen species (ROS) production (A) and comet assay on DNA damage (B) resulting from monoclonal mesenchymal stem cells according to cell culture density and cell subculture (^{∗}p < 0.05, ^{∗∗}p < 0.01, ^{∗∗∗}p < 0.005).
FIG. 9 shows the result of measuring the 8-oxo-deoxyguanosine (8-hydroxy-2'-deoxyguanosine (8-OHdG) concentration in order to confirm the degree of DNA damage caused by reactive oxygen species produced according to cell culture density and cell subculture (^{∗}p < 0.05, ^{∗∗}p < 0.01, ^{∗∗∗}p < 0.005).
FIG. 10 shows the results of confirming the change in cell proliferation ability after culturing monoclonal mesenchymal stem cells of passages 11 (P11) to 15 (P15) under the condition of only high-density (HD) or high-density + ascorbic acid (a type of antioxidant) (HD + AA).
FIG. 11 shows the results of comparing the level of reactive oxygen species produced after culturing monoclonal mesenchymal stem cells of passage 15 (P15) under the condition of only high-density (HD) or high-density + ascorbic acid (HD + AA) (^{∗}p < 0.05, ^{∗∗}p < 0.01, ^{∗∗∗}p < 0.005).
FIG. 12a shows a comparison between experimental methods of the conventional subfractionation culturing method and the improved subfractionation culturing method.
FIG. 12b shows a schematic diagram of the improved subfractionation culturing method, which is a schematic diagram showing a low-density culture corresponding to post-passage 2, which is different from the conventional subfractionation culturing method.

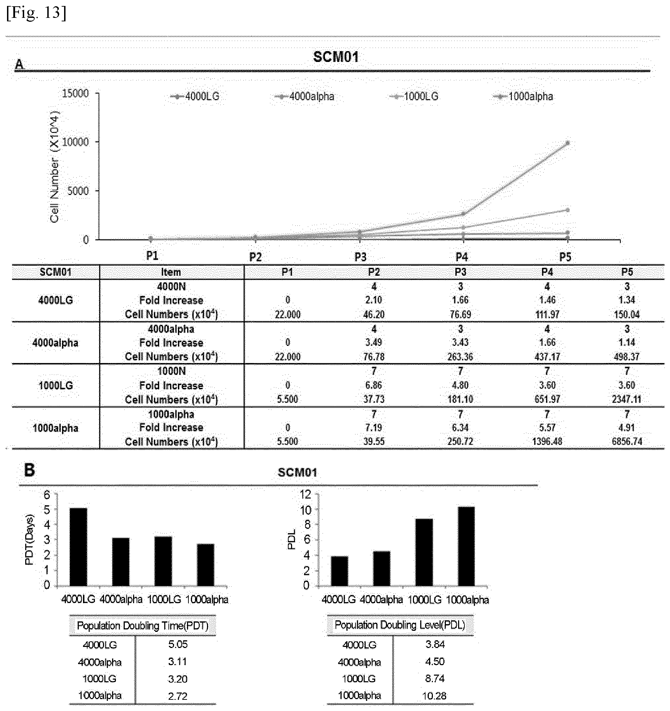

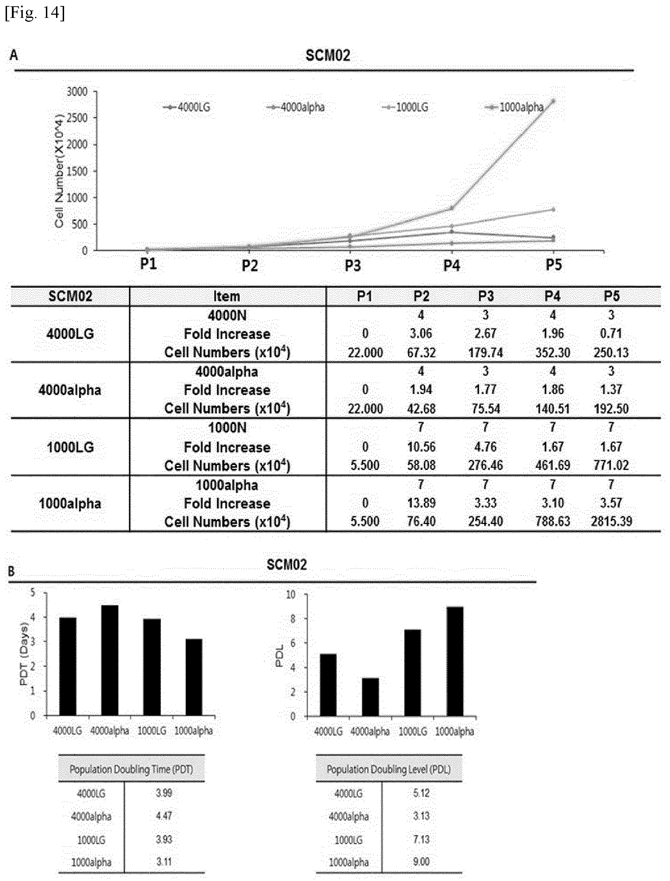

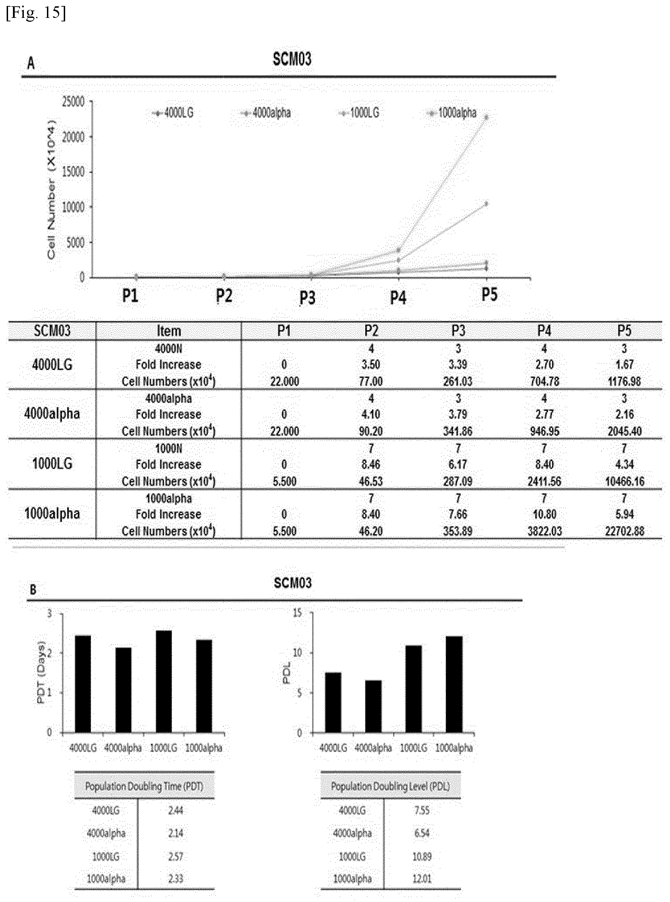

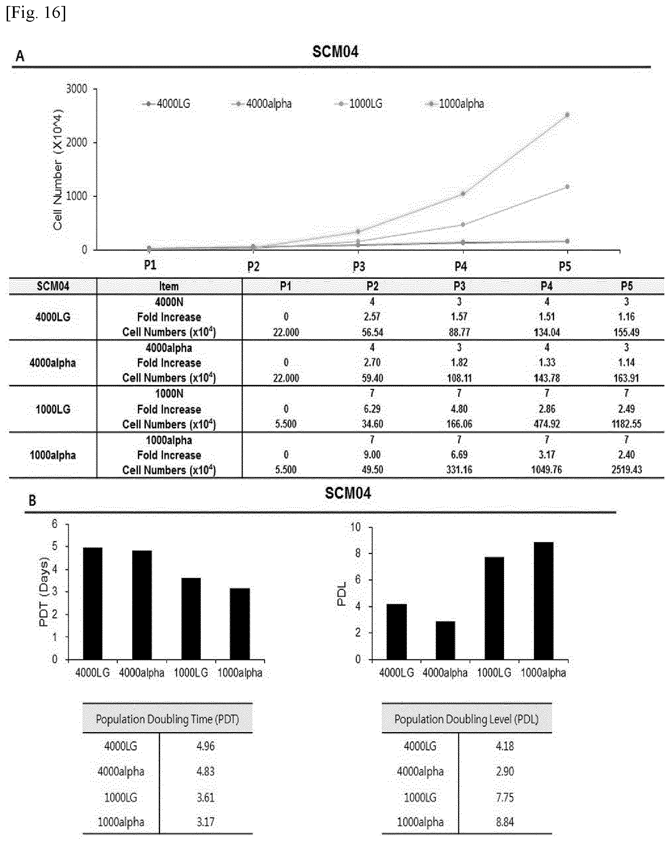

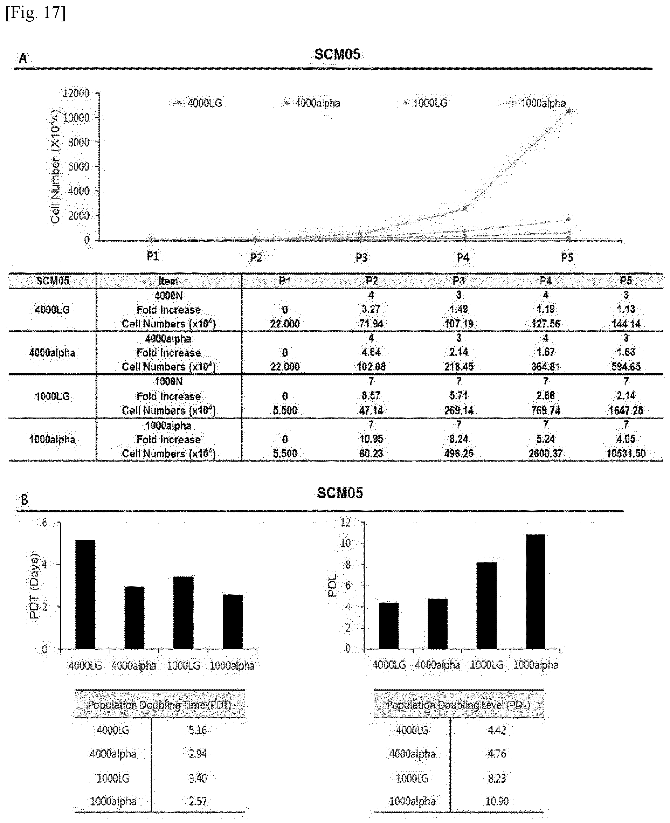

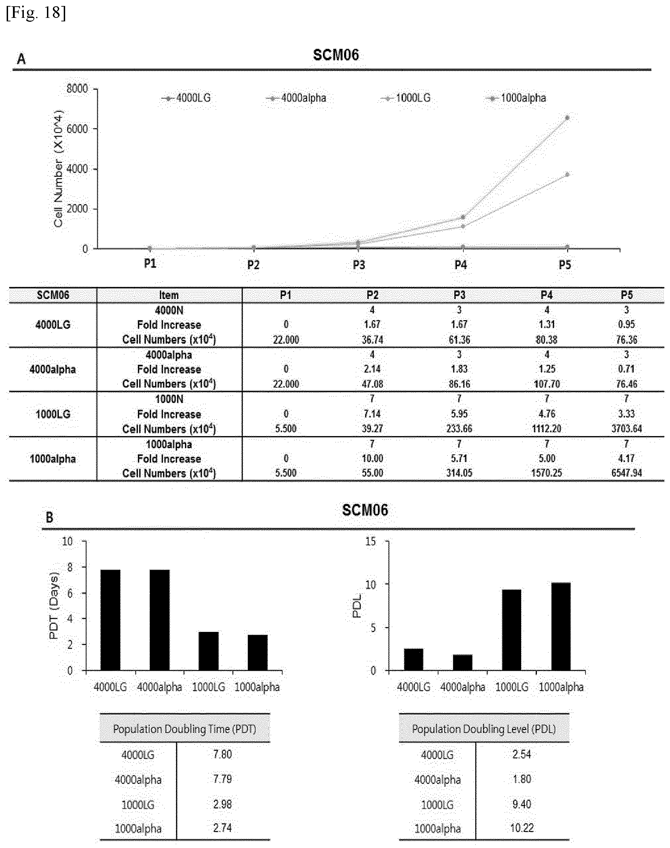

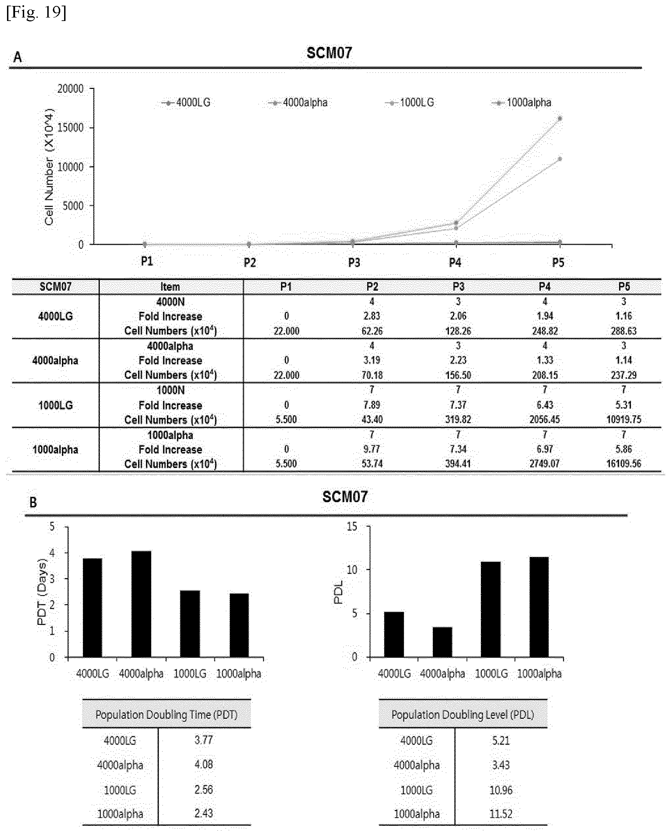

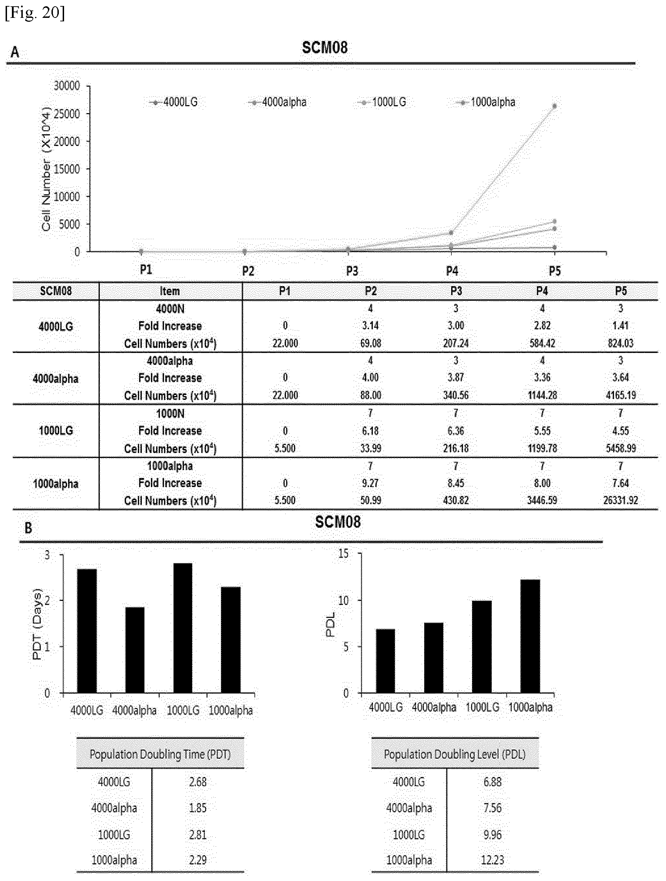

FIGS. 13 to 20 show the results of confirming the proliferation rate of cells cultured using LG-DMEM (Dulbecco's Modified Eagle Medium, low glucose) and α-MEM (Minimum Essential Medium α) with or without the addition of antioxidants after SCM01 to SCM08 monoclonal mesenchymal stem cells obtained through the subfractionation culturing method were inoculated at a density of 1,000 or 4,000 cells/cm². A in each FIG. shows the change in cell number according to passage 1 (P1) to passage 5 (P5) in each experimental group, and B in each FIG. shows the cell population doubling time (PDT) and cell population doubling level (PDL).
FIG. 21 shows the results of confirming the cell proliferation rate in the experimental group using the LG-DMEM medium without the addition of antioxidants and only changing the cell density at a density of 1,000 or 4,000 cells/cm².
FIG. 22 shows the result of confirming the cell population doubling time (PDT) and cell population doubling level (PDL) in the experimental group using LG-DMEM medium without antioxidants and only changing the cell density at a density of 1,000 or 4,000 cells/cm².
FIG. 23 shows the results of confirming the cell proliferation rate in the experimental group using α-MEM medium with the addition of antioxidants and only changing the cell density at a density of 1,000 or 4,000 cells/cm².
FIG. 24 shows the results of confirming the PDT and PDL in the experimental group using α-MEM medium with the addition of antioxidants and only changing the cell density at a density of 1,000 or 4,000 cells/cm².
FIG. 25 shows the results of confirming the cell proliferation rate in the experimental group in which the cell density was fixed at 1,000 cells/cm², and the culture medium was changed to LG-DMEM or α-MEM.
FIG. 26 shows the results of confirming the PDT and PDL in the experimental group in which the cell density was fixed at 1,000 cells/cm², and the culture medium was changed to LG-DMEM or α-MEM.
FIG. 27 shows a schematic diagram of a clinical protocol for atopic dermatitis according to the present invention.
FIG. 28 shows the results of confirming the clinical score measurement of patient A for each visit (FIG. 28a shows the results of EASI, FIG. 28b shows the results of SCORAD, and FIG. 28c shows the results of BSA).
FIG. 29 shows the results of EASI, SCORAD, BSA levels and IGA, and eosinophil count of patient A.
FIG. 30 shows the results of confirming the clinical score measurement of patient B for each visit (FIG. 30a shows the results of EASI, FIG. 30b shows the results of SCORAD, and FIG. 30c shows the results of BSA).
FIG. 31 shows the results of EASI, SCORAD, BSA levels and IGA, and eosinophil count of patient B.
FIG. 32 shows the results of confirming the score of Pruritus and Sleep, indicating the scale of itch and sleep disorder in patient B.
FIG. 33 shows the results of confirming the change of atopic-related factors in the blood of patient A according to the administration of the monoclonal stem cell of the present invention.
FIG. 34 shows the results of confirming the change of atopic-related factors in the blood of patient B according to the administration of the stem cells of the present invention.
FIG. 35 shows the results of comparing the marker expression of the patient group A and B exhibiting very excellent therapeutic effect, which was confirmed to be suitable for application of the treatment method of the present invention and the patient group exhibiting a relatively low therapeutic effect.

### [Modes of the Invention]

The present invention relates to a composition for screening atopic dermatitis patients suitable for administration of monoclonal stem cells, the composition including one or more selected from the group consisting of IL-10, IL-13, IFNg, IL-5 and IL-17; or an agent for measuring their expression.

Further, the present invention relates to a composition for predicting the prognosis of atopic dermatitis patients according to the administration of monoclonal stem cells, the composition including one or more selected from the group consisting of IL-10, IL-13, IFNg, IL-5 and IL-17; or an agent for measuring their expression.

Further, the present invention relates to a kit for screening atopic dermatitis patients suitable for administration of monoclonal stem cells, the kit including the composition for the selection or a kit for predicting the prognosis of atopic dermatitis patients according to the administration of monoclonal stem cells, the kit including the composition for prediction of prognosis.

Further, the present invention relates to a method for screening atopic dermatitis patients suitable for a method for administration of monoclonal stem cells, the method including a step of measuring the expression of one or more selected from the group consisting of IL-10, IL-13, IFNg, IL-5 and IL-17 in a sample of atopic dermatitis patient; and a method for providing information on the prognosis prediction of atopic dermatitis treatment according to the administration of monoclonal stem cells, the method including a step of measuring the expression of one or more selected from the group consisting of IL-10, IL-13, IFNg, IL-5 and IL-17 in a sample of atopic dermatitis patient.

In the present invention, the monoclonal stem cells according to the present invention are administered to atopic dermatitis patients in the required regimen, dose, and cycle, preferably at a dose of 1 × 10³ cells/kg to 1 × 10⁸ cells/kg at 1 to 3-week intervals to the individual, more preferably, for example, while repeatedly administering monoclonal stem cells in 1 to 5 cycles at a rate of 0.5 m/min to 7 m/min for 5 minutes to 15 minutes. Then, various clinical symptoms and serological indicators of atopic dermatitis patients were confirmed. The results indicate that through the measurement and detection of IL-10, IL-13, IFNg, IL-5 and IL-17 in atopic dermatitis patients, suitable atopic dermatitis patients who may exhibit excellent therapeutic effect when administered with the monoclonal stem cells of the present invention may be effectively selected and may be expected to show a reasonably good prognosis.

In the present invention, the agent for measuring the expression may be an agent for measuring the mRNA expression level or protein level of IL-10, IL-13, IFNg, IL-5 and IL-17, and an agent for measuring the mRNA expression level may be sense and antisense primers or probes that complementarily bind to the mRNA of a gene, and the agent for measuring the protein level may be an antibody that specifically binds to a protein encoded by the gene.

In the present invention, the expression "atopic dermatitis" may include congenital fever and may include allergic skin diseases whose main symptoms are dry skin and itching without limitation.

In the present invention, the expression "monoclonal stem cell of the present invention" is a stem cell having the advantages of the subfractionation culturing method that may obtain stem cells quickly and without contamination, which is obtained through an improved subfractionation culturing method that may obtain a large amount of desired monoclonal stem cells in a short time without a working cell bank (WCB) manufacturing step through the rapid proliferation of monoclonal stem cells, preferably monoclonal mesenchymal stem cells. The monoclonal stem cells obtained through the above method are characterized in that they are stem cells having an increased therapeutic effect on atopic dermatitis compared to the stem cells obtained through the conventional subfractionation culturing method.

In particular, the monoclonal stem cells used in the present invention may be obtained by the following method:
1) culturing the bone marrow isolated from the individual in a first vessel;
2) transferring only the supernatant from the first vessel to a new vessel and culturing the supernatant;
3) culturing the cells present in the new vessel and obtaining a supernatant;
4) repeating step 2) and 3) one or more times in which the supernatant of step 3) is used as the supernatant of the first vessel of step 2) to obtain monoclonal stem cells; and
5) inoculating the monoclonal stem cells of step 4) in a medium at a cell density of 50 to 1,000 cells/cm² and culturing the cells.

The culture in steps 2) and 3) is performed at 30°C to 40°C for 4 hours or less, preferably 1 hour to 3 hours, more preferably 1 hour 30 minutes to 2 hours 30 minutes, and repeated culture is performed by the culture at 30°C to 40°C for 4 hours or less, preferably 1 hour to 3 hours, more preferably 1 hour 30 minutes to 2 hours 30 minutes, followed by repeating culture two times or three times at 30°C to 40°C for 12 hours to 36 hours, preferably 18 hours to 30 hours and then culture 30°C to 40°C for 24 to 72 hours or 36 hours to 60 hours, preferably 36 hours to 60 hours. Each time it may be carried out by moving the supernatant to a new culture vessel.

The separation method in Example of the present invention is briefly summarized as follows.

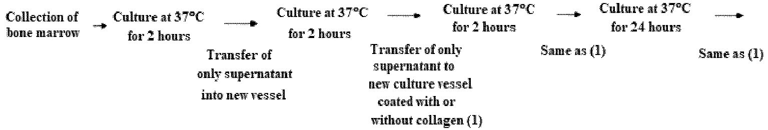

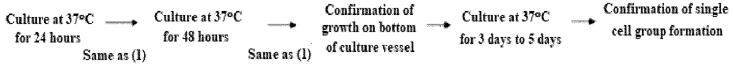

Cultured cells form a monoclonal cell group. After separating the monoclonal cell group, the subculture may be performed. The present invention includes the subculture of step 5) in addition to the conventional sub fractionation culturing method.

In the present invention, the expression "subfractionation culturing method (SCM)" means a method of separating stem cells by specific gravity. It refers to a process of repeating the following process several times in which first, human bone marrow is extracted and cultured in a cell culture medium, then only the supernatant is collected and transferred to a culture vessel treated with or without a coating agent and it is cultured. Such subfractionation culturing is characterized by repeating a process of obtaining and culturing the supernatant without a centrifugation process. Finally, there is an advantage in that monoclonal stem cells, preferably monoclonal mesenchymal stems can be obtained without contamination of other cells.

Steps 1) to 4) of steps 1) to 5) of the present invention may be performed the same or equivalent to the subfractionation culturing method described in Korean patent application No. 10-2006-0075676 or Korean patent application No. 10-2014-0170045, and Korean patent application No. 10-2006-0075676 is incorporated herein by reference in its entirety.

Further, Korean patent application No. 10-2014-0170045 as conventional art, discloses a method of obtaining cells in relation to the treatment of atopic dermatitis. The method relates to a method for preventing or treating atopic dermatitis, including steps of: (i) culturing a sample containing bone marrow-derived mesenchymal stem cells in a first vessel to obtain a supernatant; (ii) transferring the supernatant from the first vessel to the second vessel; (iii) culturing the cells present in the second vessel to obtain a supernatant; (iv) repeating steps (ii) and (iii) three or more times; (v) isolating single cell-derived colonies; and (vi) transferring the cells from the colony to the growth medium, and culturing the cells; to obtain a clonal stem cell obtained through a subfractionation culturing method to use the same. This is a method of obtaining monoclonal stem cells only with a difference in density without centrifugation, and thus it uses the conventional subfractionation culturing method of Korean patent application No. 10-2006-0075676.

However, the subfractionation culturing method of Korean patent application No. 10-2006-0075676 and Korean patent application No. 10-2014-0170045 do not include a method of obtaining monoclonal stem cells in which monoclonal stem cells are effectively obtained at a low passage, and through this, the therapeutic effect on atopic dermatitis is remarkably improved.

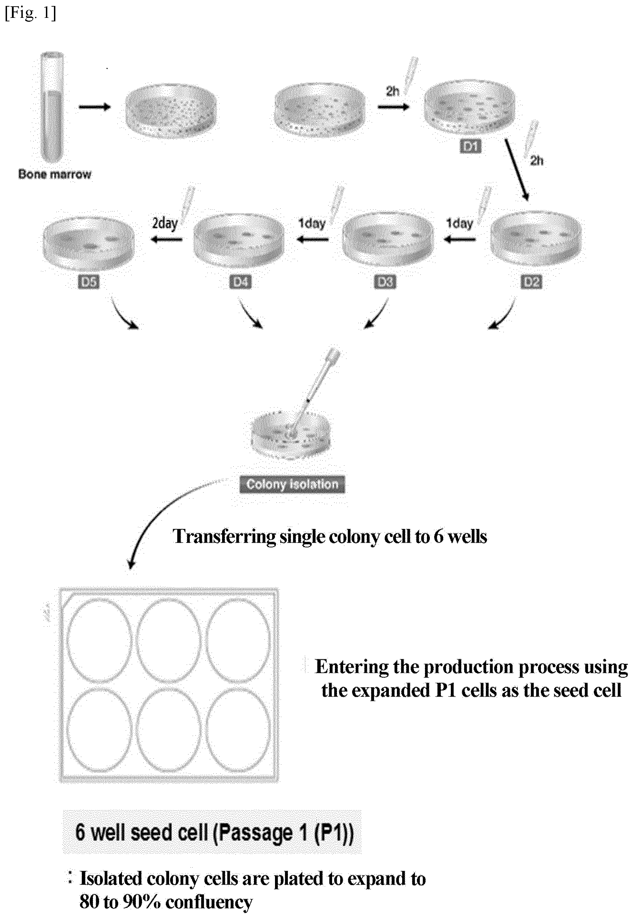

As shown in FIG. 1, the conventional subfractionation culturing method is performed such that all cells obtained from a single colony are transferred to 6-well, they are proliferated in confluency of 80% to 90%, and then proliferated passage 1 (P1) cells are cultured as seed cells at high-density of 4,000 cells/cm² in order to obtain many cells without recognition of density control.

On the other hand, the present invention relates to an "improved subfractionation culturing method" based on the fact that stem cells having excellent effects in preventing, treating, and improving atopic dermatitis may be effectively obtained by controlling the cell density in culture after passage 2. It is characterized in that the improved subfractionation culturing method has the culture step after the seed cell, which is different from that of the conventional subfractionation culturing method. For example, specifically, it includes a step of "5) inoculating the monoclonal stem cells of step 4) into a medium at a cell density of 50 to 1,000 cells/cm² and culturing." Since the improved subfractionation culturing method may induce rapid proliferation of monoclonal stem cells compared to the conventional subfractionation culturing method, the final product may be obtained quickly, and preferably only culture less than passage 10 such as P2 to P8 allows the production of master cell bank (MCB) to obtain monoclonal stem cells exhibiting excellent atopic dermatitis prevention or therapeutic effect.

When the monoclonal stem cells of the present invention are cultured at a high density of 4,000 cells/cm² as in the conventional process, the cell proliferation ability is significantly reduced, the markers of the mesenchymal stem cells are changed, and the differentiation ability of the stem cells may be lost. Therefore, the monoclonal stem cells obtained through the improved subfractionation culturing method may mean cells cultured at a low to medium cell density such as low cell density of 4,000 cells/cm² or less, for example, 3,000 cells/cm² or less, preferably 2,000 cells/cm² or less, more preferably 50 to 1,000 cells/cm².

When monoclonal mesenchymal stem cells are cultured at a cell density of 1,000 cells/cm² or less, the proliferation ability of the cells is maintained at a remarkably high level throughout the long incubation period compared to mesenchymal stem cells cultured at a high density such as 4,000 cells/cm², so there is an advantage in that the desired amount of mass monoclonal cells may be quickly obtained without repeating many passages. Therefore, the improved subfractionation culturing method of the present invention may be characterized in that the subculture step after the seed cell is performed only at less than passage 10, preferably less than passage 8. Thus, it is possible to mass-produce monoclonal stem cells with only a small number of passages compared to the need to culture up to passages 25 to secure a sufficient number of cells in the conventional subfractionation culturing method.

Further, when monoclonal mesenchymal stem cells are cultured at the above cell density, there are advantages that the cells have little DNA damage, aging is suppressed to effectively maintain the differentiation ability of stem cells, thereby quickly and speedily obtaining monoclonal mesenchymal stem cells having excellent stem cell characteristics.

In addition, the monoclonal stem cells obtained according to the method of the present invention exhibit excellent prevention, improvement or therapeutic effect on atopic dermatitis compared to the monoclonal stem cells cultured at a high density such as 4,000 cells/cm².

The medium used in the present invention may include both a medium without an antioxidant, a medium in which an antioxidant is added to the medium, or a medium with an antioxidant.

The medium without antioxidants is not limited thereto, but DMEM medium may be used, and if necessary, culture may be performed by further adding antioxidants to the medium. In addition, if necessary, culture may be performed using α-MEM medium with antioxidants.

The antioxidant of the present invention may include, without limitation, antioxidants that can be used in cell culture, for example, one or more selected from the group consisting of glutathione, cysteine, cysteamine, ubiquinol, betamercaptoethanol and ascorbic acid (AA). When the antioxidant is added to the medium, the antioxidant may be added at a concentration of 10 µg/ml to 50 µg/ml, preferably 10 µg/ml to 30 µg/ml, more preferably 25 µg/ml.

In one Example of the present invention, DMEM, more preferably LG-DMEM medium is used as a medium without antioxidants, and α-MEM medium is used as a medium with ascorbic acid as an antioxidant.

Meanwhile, according to the method of the present invention, the monoclonal stem cells may be proliferated very effectively, thereby excluding a process of producing a working cell bank (WCB) using MCB. This simplifies the process compared to the conventional subfractionation culturing method that WCB should be prepared after MCB is prepared.

When using a medium with an antioxidant as the culture medium of the present invention, gentamicin may be added as an antibiotic to the culture medium.

The mesenchymal stem cells obtained by the method of the present invention are finally preferably P2 to less than P10 mesenchymal stem cells, more preferably P2 to P8 mesenchymal stem cells, even more preferably P2 to P6 mesenchymal stem cells.

This is a stem cell obtained at a lower passage than the conventional process in which mesenchymal stem cells of at least P10 to P12 are obtained as a final product. Mesenchymal stem cells rapidly proliferated at a low passage through cell inoculation density control may be easily obtained in large quantities.

Compared to monoclonal stem cells obtained by improved subfractionation culturing method as described above at a low density of preferably 2,000 cells/cm² or less, more preferably 1,000 cells/cm² or less under antioxidative conditions, the present invention is excellent in the effect of smaller and homogeneously forming cell size, alleviating the thickening of the dermis or epidermis induced by atopic dermatitis, suppressing the production of at least one selected from the group consisting of IgE, IgG1 and IL-4, promoting the production of INF-γ and inhibiting mast cells.

In the present invention, screening of atopic dermatitis patients suitable for monoclonal stem cell administration of the present invention or prognosis prediction of atopic dermatitis patients according to monoclonal stem cell administration may be performed as follows.

First, the expression of one or more selected from the group consisting of IL-10, IL-13, IFNg, IL-5 and IL-17 is measured or detected in a sample of atopic dermatitis patients. When the patient's i) IL-10 is 20 pg/ml or more and IL-13 is 100 pg/ml or more, ii) IFNg and IL-5 are not detected, and/or iii) IL-17 is detected, the patient is selected as a patient suitable for monoclonal stem cell administration method or information that when monoclonal stem cells are administered to the patient, an excellent treatment prognosis for atopic dermatitis will appear may be provided.

In the present invention, for the method for measuring or detecting the expression of one or more selected from the group consisting of IL-10, IL-13, IFNg, IL-5 and IL-17, methods commonly used in the art may be used without limitation, but preferably may be ELISA.

In the present invention, the expression of IL-10 in a patient suitable for administration of monoclonal stem cells may be 20 pg/ml or more, preferably 20 pg/ml to 500 pg/ml, and the expression of IL-13 is 100 pg/ml or more, preferably 100 pg/ml to 4,000 pg/ml.

In the present invention, the sample may be selected from the group consisting of blood, serum, plasma, lymph, cerebrospinal fluid, ascites, urine and tissue biopsy of atopic dermatitis patients.

When a patient is selected as one suitable for the monoclonal stem cell administration method through the method as described above or a patient is confirmed to have an excellent treatment prognosis, atopic dermatitis may be treated by administering the monoclonal stem cells of the present invention. For example, one dose is 1 × 10³ cells/kg to 1 × 10⁸ cells/kg, and it is administered at intervals of 1 week to 3 weeks. Preferably, the administration is performed as 1 cycle, which is composed of 2 to 5 times of the above administration. Further, depending on the condition of the patient, 2 to 5 cycles of administration may be repeated. Further, the administration may be preferably carried out by intravenous administration. In the case of treatment by intravenous administration, it may be preferable to administer for 5 minutes to 15 minutes at a rate of 0.5 m/min to 7 m/min.

In the most preferred embodiment of the present invention, a total of 3 administrations with an interval of 2 weeks is set as 1 cycle, and depending on the patient's condition and degree of improvement, it may be administered repeatedly in 2 to 5 cycles.

Accordingly, the present invention may provide a pharmaceutical composition for preventing or treating atopic dermatitis, and in particular, the composition may be used for the treatment of a patient selected as suitable for monoclonal stem cell treatment.

Accordingly, the present invention provides a pharmaceutical composition for preventing or treating atopic dermatitis, the composition including monoclonal stem cells, which is for administration when before treatment, the patient's i) IL-10 is 20 pg/ml or more or IL-13 is 100 pg/ml or more, ii) IFNg and IL-5 are not detected, or iii) IL-17 is detected.

In particular, monoclonal stem cells included in the pharmaceutical composition of the present invention are preferably monoclonal stem cells obtained by steps of 1) culturing the bone marrow isolated from the individual in a first vessel; 2) transferring only the supernatant from the first vessel to a new vessel and culturing the supernatant; 3) culturing the cells present in the new vessel and obtaining a supernatant; 4) repeating step 2) and 3) one or more times in which the supernatant of step 3) is used as the supernatant of the first vessel of step 2) to obtain monoclonal stem cells; and 5) inoculating the monoclonal stem cells of step 4) in a medium at a cell density of 50 to 1,000 cells/cm² and culturing the cells to obtain monoclonal stem cells.

Further, the present invention provides a method for treating atopic dermatitis, the method including steps of 1) culturing the bone marrow isolated from the individual in a first vessel; 2) transferring only the supernatant from the first vessel to a new vessel and culturing the supernatant; 3) culturing the cells present in the new vessel and obtaining a supernatant; 4) repeating step 2) and 3) one or more times in which the supernatant of step 3) is used as the supernatant of the first vessel of step 2) to obtain monoclonal stem cells; 5) inoculating the monoclonal stem cells of step 4) in a medium at a cell density of 50 to 1,000 cells/cm² and culturing the cells to obtain monoclonal stem cells; and 6) administering the obtained monoclonal stem cells to an individual at a dose of 1 × 10³ to 1 × 10⁸ cells/kg at an interval of 1 week to 3 weeks.

In the method and composition for preventing or treating atopic dermatitis according to the present invention, the monoclonal stem cells may be, for example, bone marrow-derived mesenchymal stem cells.

When the stem cells are used for the purpose of preventing, treating, or improving atopic dermatitis, the stem cells may be used without limitation in the form of fresh or frozen type. The fresh type may be administered with a longer administration cycle, and the frozen type may be administered with a shorter administration cycle than the fresh type.

The monoclonal mesenchymal stem cells according to the present invention may be administered to an individual at a dose of 1 × 10³ to 1 × 10⁸ cells/kg, for example, may be administered to an individual total 2 to 5 times at an interval of 1 week to 3 weeks, most preferably, may be administered to the patient in a cycle of total 3 times at an interval of 2 weeks at a dose of 1 × 10⁶ cells/kg. The cycle may be repeated 1 to 5 times, depending on the condition of the patient.

Further, the present invention provides a kit for preventing or treating atopic dermatitis for the purpose of effective administration according to the administration regimen, dose, and cycle according to the present invention.

The kit may be a kit containing 1 to 10 compartments for the use of appropriate usage and dose in which one compartment contains stem cells at a concentration of 1 × 10³ to 1 × 10⁸ cells/kg as basic configuration, but the form is not limited thereto. For example, the kit may include 3 compartments for 3 administrations and 10 compartments for 10 administrations.

The monoclonal stem cells of the present invention may be administered to an induvial through various routes for stem cell therapy. Preferably, it may be administered intravenously. When administered intravenously, the speed may be appropriately adjusted depending on the patient's weight, health status, and disease state, but for example, it may be administered at a speed of 0.5 m/min to 10 m/min, 0.5 m/min to 7 m/min for 5 minutes to 15 minutes.

In the treatment method, composition and kit of the present invention, the stem cells are preferably monoclonal stem cells obtained by steps of 1) culturing the bone marrow isolated from the individual in a first vessel; 2) transferring only the supernatant from the first vessel to a new vessel and culturing the supernatant; 3) culturing the cells present in the new vessel and obtaining a supernatant; 4) repeating step 2) and 3) one or more times in which the supernatant of step 3) is used as the supernatant of the first vessel of step 2) to obtain monoclonal stem cells; and 5) inoculating and culturing the monoclonal stem cells of step 4) in a medium at a cell density of 50 to 1,000 cells/cm² and obtaining monoclonal stem cells.

The stem cells may also be monoclonal stem cells obtained by culturing the cells in a medium of step 5) to which antioxidants are added and may be monoclonal stem cells obtained by the culture in step 5) using passages 2 to 8.

Atopic dermatitis individuals suitable for the treatment method of the present invention may be selected by measuring one or more selected from the group consisting of IL-10, IL-13, IFNg, IL-5 and IL-17 and their expression. They may be used to predict their prognosis.

Therefore, the individual to be administered the composition for the prevention or treatment of atopic dermatitis of the present invention or the individual to be treated with the atopic dermatitis treatment method of the present invention is preferably one characterized in that before treatment, the expression of IL-10 is 20 pg/ml or more and the expression of IL-13 is 100 pg/ml or more, IFNg and IL-5 are not expressed, or IL-17 is expressed.

In the present invention, the "individual" includes an individual in need of prevention or treatment of atopic dermatitis and may be a mammal or a mammal other than a human.

When monoclonal stem cells of the present invention are administered to an individual, it may be administered in combination with a drug for preventing or treating atopic dermatitis known in the art, and it may be administered in a formulated form together with excipients for stem cell therapeutics known in the art that are approved or confirmed to be harmless to the human body.

The monoclonal stem cell of the present invention may effectively alleviate the symptoms of percutaneous and dermal thickening and keratinization appearing in atopic dermatitis-induced skin, and in particular, may be characterized in alleviating the thickening of the dermis or epidermis.

Further, the monoclonal stem cells of the present invention may improve various immune and inflammatory factors induced in atopic dermatitis, preferably reduce IgE, IL-18, and IL-13, and effectively reduce IL-17 known as markers that increase in dry lesions. Further, using a Th2 chemokine, they may reduce IL-22, which causes epidermal hyperproliferation and the blood levels of CCL17 and CCL22, which are biomarkers reflecting the diagnosis and severity of allergic diseases.

The monoclonal stem cells obtained by the method of the present invention may be characterized in that the improvement effect of histological and physiological factors related to atopic dermatitis is excellent compared to the stem cells (GCM-MSC) obtained by the conventional density gradient centrifugation method as well as the clonal stem cells obtained by the conventional subfractionation culturing method.

In addition, the method and composition for preventing or treating atopic dermatitis using monoclonal stem cells of the present invention may effectively improve EASI, SCORAD, BSA levels and IGA, and eosinophil levels for evaluating the clinical severity of atopic dermatitis and be effective in improving sleep disturbance and itching accompanying atopic dermatitis patients.

In order to administer the pharmaceutical composition of the present invention, the pharmaceutical composition of the present invention may be prepared by including one or more pharmaceutically acceptable carriers in addition to the active ingredient for administration. Pharmaceutically acceptable carriers included in the pharmaceutical composition of the present invention are those commonly used in formulation, for example, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methylcellulose, methyl hydroxybenzoate, propyl hydroxybenzoate, talc, magnesium stearate and mineral oil, and the like but is not limited thereto. The pharmaceutical composition of the present invention may further include a lubricant, a wetting agent, a sweetening agent, a flavoring agent, an emulsifying agent, a suspending agent, a preservative, and the like, in addition to the above components.

The dose of the pharmaceutical composition of the present invention may vary depending on the formulation method, administration mode, administration time and/or route of administration of the pharmaceutical composition, and vary depending on similar factors well known in the medical field and several factors such as type and degree of response to be achieved by the administration of the pharmaceutical composition, the type, age, weight, general health condition, symptoms or severity of disease, gender, diet, excretion of the individual to be administered, drug or other composition used concurrently with the individual or for transplantation. A person skilled in the art can easily determine and prescribe an effective dosage for a desired treatment.

The administration route and administration mode of the pharmaceutical composition of the present invention may be independent of each other. The method is not particularly limited thereto. Any route and mode of administration may be included as long as the pharmaceutical composition may reach the desired site.

The pharmaceutical composition may be administered by oral administration or parenteral administration. The parenteral administration method includes, for example, intravenous administration, intraperitoneal administration, intramuscular administration, transdermal administration, or subcutaneous administration. Methods of applying, spraying, or inhaling the pharmaceutical composition to the diseased site may also be used. However, the pharmaceutical composition is most preferably administered by intravenous administration.

In the treatment method and preparing method of the present invention, the contents described in the composition above may be equally applied thereto, and overlapping contents are excluded in order to avoid the complexity of the description of the specification.

Hereinafter, the present invention is described in detail with Examples.

The following Examples are intended to illustrate the present invention, but the content of the present invention is not limited to the following Examples.

### Example 1. Establishment of improved subfractionation culturing method

The improved mesenchymal stem cell subfractionation culturing method and proliferation method were used to prepare monoclonal mesenchymal stem cells exhibiting excellent effects on atopic dermatitis. The improved mesenchymal stem cell subfractionation culturing method and proliferation method are characterized by changing the cell density and culture medium among the culture conditions of the subfractionation culturing method described in Korean Patent Application No. 10-2006-0075676. In the experiments as below, the cell culture densities of the monoclonal mesenchymal stem cells (cMSCs) obtained by the subfractionation culturing method were varied in 50 cells/cm² (low density), 1,000 cells/cm² (medium density) and 4,000 cells/cm² (high density), thereby analyzing the characteristics of the cells.

### 1.1 Confirmation of morphological changes of mesenchymal stem cells according to cell density

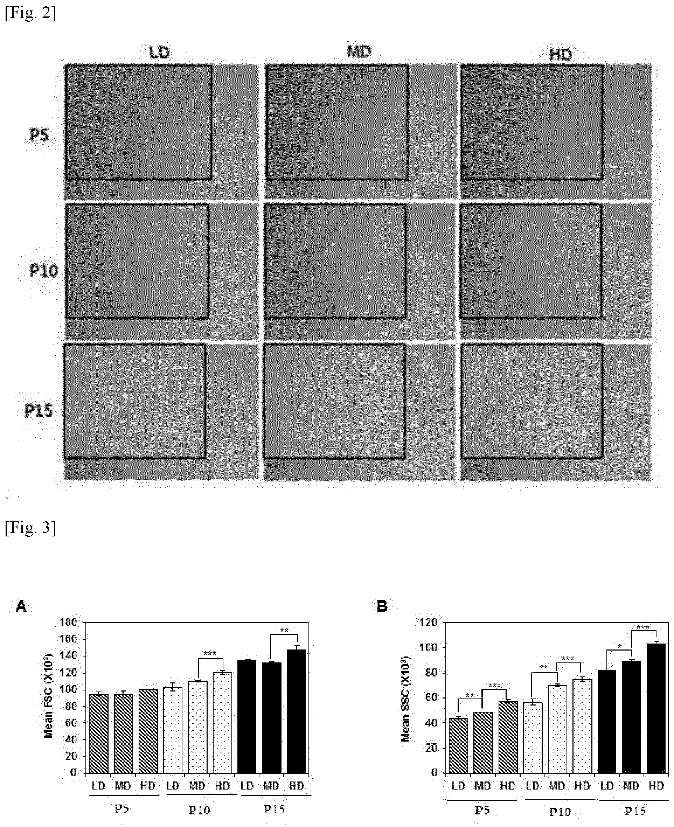

First, the experiments were conducted to confirm the morphological changes of mesenchymal stem cells according to cell density in long-term culture. The MSCs having passage 5 (P5), passage 10 (P10) and passage 15 (P15) were used for the long-term culture conditions. The mesenchymal stem cells were inoculated in LG-DMEM under the condition of low density, medium density and high density, respectively. Thereafter, the morphological changes of the cells were observed through a microscope to determine whether or not the stem cells were aged. The results are illustrated in FIG. 2.

As illustrated in FIG. 2, the cell size and morphological pattern of P5 and P10 were different according to the cell density. In particular, P15 cultured under a high density culture condition had flat and enlarged mesenchymal stem cells. This morphology form indicates the typical aging of mesenchymal stem cells, which confirmed that the cell density is controlled in the long-term culture, resulting in the control of mesenchymal stem cells' aging.

### 1.2 Confirmation of MSC size and granularity according to cell density

In order to further confirm the change of stem cells according to the cell density, the cell size and granularity, which were known to be increased in aged cells, were analyzed by FACS analysis. Thus, the results are illustrated in FIG. 3.

As illustrated in FIG. 3, the cell size did not show a significant difference at P5, but P10 and P15 showed significant differences according to the cell density. In particular, the cell sizes in P10 and P15 were significantly increased under a culture condition of the high cell density, thereby further promoting the aging of cells. Similarly, the cell granularity also was significantly increased as the cell density was increased in all passages.

Therefore, it was confirmed that controlling the cell density of mesenchymal stem cells in long-term culture may be a factor to control aging of cells. Further, the cell culture density is lower to improve the morphological changes in the late passage.

### 1.3 Confirmation of aging of mesenchymal stem cells according to culture cell density

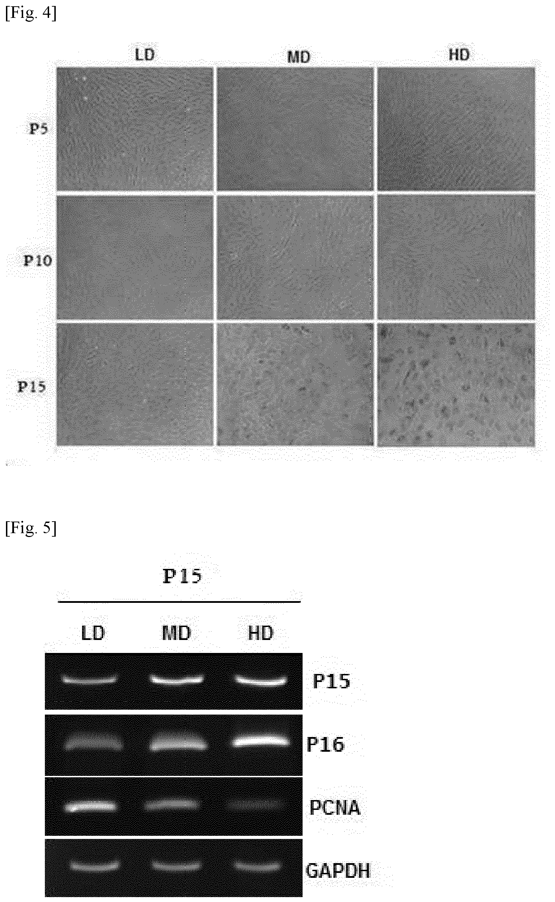

The beta-galactosidase-staining analysis was performed to confirm whether the morphological changes confirmed in Examples 1.1 and 1.2 were actually an age-dependent phenomenon of mesenchymal stem cells, which can selectively stain aging cells, and RT-PCR was carried out to compare the expression of aging-related genes P15 and P16 and a proliferation marker PCNA gene. The results are illustrated in FIGS. 4 and 5, respectively.

As illustrated in FIG. 4, passage 5 (P5) and passage 10 (P10) did not have the staining of aged cells at all cell densities, but passage 15 (P15) had the staining of aged cells markedly increased as the cell density increased. As illustrated in FIG. 5, in passage 15 (P15), gene expression of CDK inhibitors p15 and p16, which are genes related to aging, was increased as the cell density increased, and PCNA, which is a proliferation marker, decreased.

These results demonstrate that the morphological changes of mesenchymal stem cells are related to the aging of mesenchymal stem cells and that the manipulation of the cell density may control the aging of mesenchymal stem cells.

### 1.4 Confirmation of change of proliferation ability of mesenchymal stem cells according to culture cell density

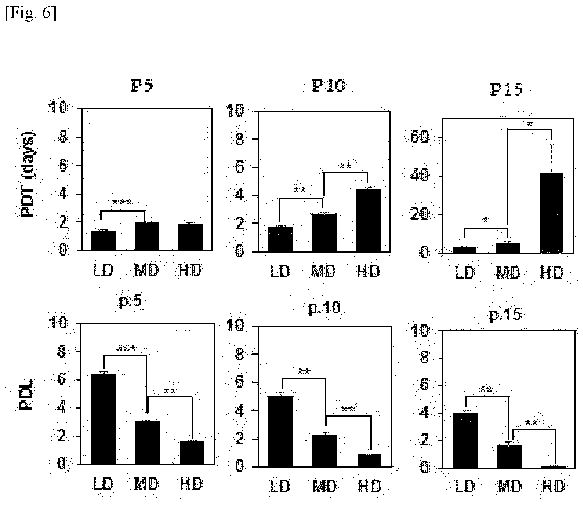

It is known that the proliferation ability of mesenchymal stem cells progressively decreases as cells are subcultured and aged. Therefore, the proliferation ability may be used as a criterion for confirming the aging of mesenchymal stem cells. Thus, the proliferation ability of mesenchymal stem cells according to the cell culture density was compared during long-term cell culture. The proliferation ability of each cell was determined by calculating the proliferation rate according to each passage using the number of cells which were initially inoculated and the number of cells that were obtained after the culture. The results are shown in Table 1 and FIG. 6.

**[Table 1]**

| **Fold Increase** | | | |
|---|---|---|---|
| **(cell/cm²)** | **P5** | **P10** | **P15** |
| **50** | 88.4±6.5 | 34.3±5.0 | 16.4±1.3 |
| **1000** | 8.5±0.3 | 4.9±0.5 | 3.1±0.4 |
| **4000** | 3.0±0.1 | 1.9±0.1 | 1.1±0.1 |

As shown in Table 1, the fold increases were 88.4, 34.3 and 16.4 at P5, P10 and P15, respectively, in mesenchymal stem cells cultured at a low density. Meanwhile, the fold increases in MSCs cultured at a medium density were 8.5, 4.9 and 3.1 at P5, P10 and P15, respectively. Further, the fold increases were 3.0, 1.9, and 1.1 at P5, P10 and P15, respectively, in mesenchymal stem cells cultured at a high density. As illustrated in FIG. 6, the cell population doubling time (PDT) and the cell population doubling level (PDL) also have the same pattern as the fold increase. These results indicate that the proliferation ability of mesenchymal stem cells may be maintained by lowering the cell density in long-term mesenchymal stem cell culture and that even though performing the same subculture, the aging of mesenchymal stem cells may be inhibited, and the lifespan of mesenchymal stem cells may be prolonged.

### 1.5 Confirmation of change of differentiation ability of mesenchymal stem cells according to culture cell density

The differentiation abilities according to P5 to P15 cultures were compared to confirm whether culture cell density affects the ability of stem cells. The ability of stem cells to differentiate into adipocytes and osteocytes was confirmed. Qualitative and quantitative analyzes were carried out at each passage and density. Specifically, NCS (Newborn Calf Serum) (Gibco), 10⁻⁷ mol dexamethasone (Sigma), 0.5 mM IBMX (Sigma), 10 µg/ml insulin (Sigma), and 100 µM indomethacin (Sigma) were added to a high glucose DMEM culture medium to prepare the adipocyte differentiation medium, and then the experiment was carried out. After 7 days of differentiation, it was confirmed by Oil red O histochemical staining. After the Oil red O histochemical staining, it was eluted with isopropyl alcohol and measured at 500 nm and quantitatively analyzed.

The osteoclast differentiation medium was prepared and used by adding FBS (Gibco), 50 µg/ml ascorbic 2-phosphate (Sigma), 10⁻⁸ mol dexamethasone (Sigma) and 10 mM β-glycerophosphate (Sigma) to α-MEM.After 21 days of differentiation, it was confirmed by Alizarin red S histochemical staining. After Alizarin red S histochemical staining, it was eluted with 10% acetic acid, measured at 405 nm and quantitatively analyzed. The adipocyte differentiation ability and the osteoclast differentiation ability were confirmed as described above. The results are illustrated in FIG. 7.

As illustrated in FIG. 7, the adipocyte differentiation ability decreased overall as the passage progressed, but the difference according to the density was not apparent. On the other hand, the osteoclast differentiation ability significantly decreased in the P15 culture group under the condition of high density. These results show that the osteoclast differentiation ability of mesenchymal stem cells may be maintained better when culturing at a low cell density.

### 1.6 Antigen profile analysis of mesenchymal stem cells according to culture cell density

Experiments were carried out to confirm whether or not the cell culture density also affects the stem cell antigen expression. Flow cytometry was performed to confirm the changes in positive and negative antigen expression according to each passage and culture density. The results are shown in Table 2

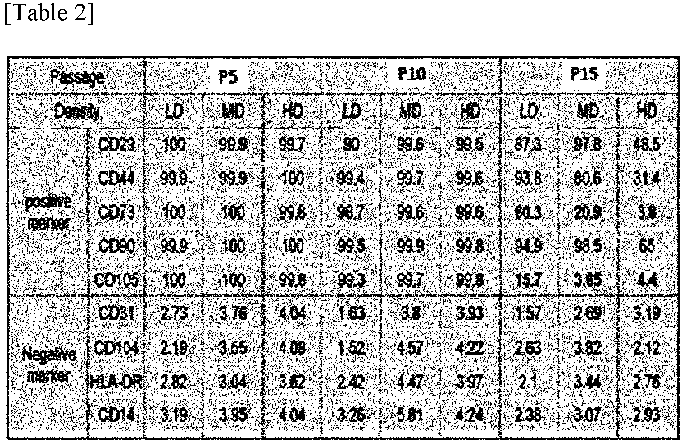

As shown in Table 2, the change of the expression of the negative marker was not clearly apparent, but the expression level of some positive markers was changed according to the cell culture density even in the same passage.

In particular, when cells were cultured at a high density in P15, the expression level of most positive markers was significantly decreased. Further, CD73 and CD 105 showed negative expressions. Thus, cell culturing with a low cell density may be a critical factor.

### 1.7 Comparison of ROS production and DNA damage according to culture cell density

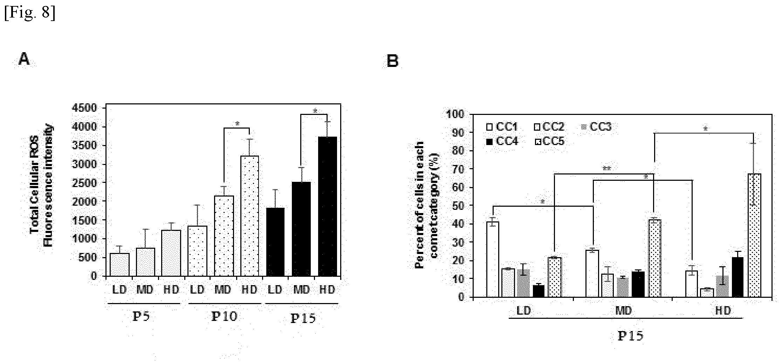

It is known that the decrease of mesenchymal stem cell function is associated with DNA damage. In particular, DNA damage induced by ROS, which is an active oxygen species, is known to promote the aging of mesenchymal stem cells (MSCs). Therefore, in order to confirm whether or not total ROS production and DNA damage thereby are different according to culture density, fluorescence intensity analysis was performed to compare the total amount of cellular ROS according to the passage and cell culture density. Comet analysis was performed to confirm the degree of DNA damage. The results are illustrated in FIG. 8.

As illustrated in FIG. 8, total ROS production tended to increase as the cell culture density increased in all passages. In particular, ROS production significantly increased at P10 and P15 (See FIG. 8A). In the comet analysis, the data were classified from CC1 with the weakest DNA damage to CC5 with the most severe DNA damage. The CC5 with the most severe DNA damage exhibited a significant increase as the cell culture density increased. On the other hand, the CC1 tended to decrease significantly as the cell density increased (See FIG. 8B).

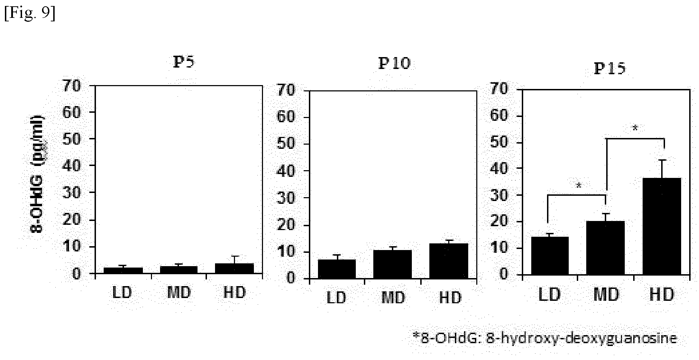

Further, in order to confirm whether or not ROS caused DNA damage, an experiment was conducted to confirm the concentration of 8-OHdG which may identify DNA damage caused by ROS. The analysis of 8-OHdG is as follows. 50 µl of the DNA sample collected from each cell was placed on an 8-OHdG conjugate-coated plate and cultured at room temperature for 10 minutes. Then, an anti-8-OHdG antibody was added thereto, and the mixture was cultured at room temperature for 1 hour. After washing three times, the secondary antibody-enzyme conjugate was added to each well, and the mixture was cultured at room temperature for another 1 hour. After washing three times, a substrate solution was added thereto, and the mixture was cultured at room temperature for 30 minutes. Finally, a stop solution was added thereto. The absorbance intensity thereof was measured at 450 nm. The results are illustrated in FIG. 9.

As illustrated in FIG. 9, as the cell culture density increased, the concentration of 8-OHdG was significantly increased in the P15 group in which DNA damage was most severe. These results demonstrate that ROS produced under the culture condition of the high density caused DNA damage to increase, thereby promoting the aging of mesenchymal stem cells.

These results show that lowering the cell culture density may play a role in protecting mesenchymal stem cells from DNA damage which is caused by increased ROS production of mesenchymal stem cells.

### 1.8 Confirmation of mesenchymal stem cell proliferation and ROS production ability according to antioxidant treatment

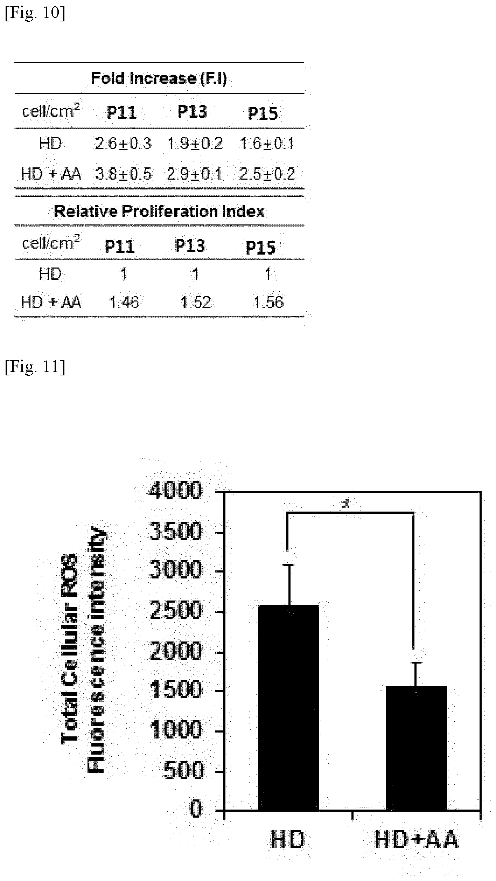

In order to confirm whether or not ROS produced under the culture condition of high density affects the proliferation of mesenchymal stem cells, an experiment for eliminating ROS was performed. 25 µg/ml of ascorbic acid, an antioxidant, was added to the culture medium under the culture condition of high density in P11 to P15. Then, the fold increase of proliferation rate was compared between the two groups. The results are illustrated in FIG. 10.

As illustrated in FIG. 10, the fold increase was 2.6, 1.9, and 1.6 at P11 to P15 in the high density culture condition. As passage number increased, the proliferation ability decreased, and as a result, aging began. However, treating with the antioxidant induced to maintain high proliferation ability at about 50% in all passages. In the antioxidant treatment group, the growth fold increase was 3.8, 2.9 and 2.5 in P11 to P15, respectively. The proliferation ability was maintained high even at P15.

At the endpoint P15, ROS levels were confirmed between the high density culture condition alone and high density culture condition + antioxidant treatment groups. The results are illustrated in FIG. 11.

As illustrated in FIG. 11, the ROS level also decreased under the condition that proliferation was increased by treating ascorbic acid, an antioxidant. Therefore, mesenchymal stem cell culture was preferably performed at a low cell density rather than a high density. ROS production induced by a high density of cell culture was eradicated with an antioxidant, thereby resulting in an increase of mesenchymal stem cell proliferation ability. In other words, ROS at a high density inhibited the proliferation ability of mesenchymal stem cells. As the cell density was lower, the ROS production was decreased, and the proliferation ability of mesenchymal stem cells was promoted.

In conclusion, these results indicate that controlling the cell density at the density of 1,000 cells/cm² or less in culture conditions is important to maintain the proliferation, culture and stem cell ability of the monoclonal mesenchymal stem cells obtained through the subfractionation culturing method. Culture with an antioxidant inhibits oxidative stress induced by cell culture, thereby promoting mesenchymal stem cell proliferation efficiently. In addition, when comparing the culture under the same low cell density condition, the stem cells at passage 10 or more, such as P15, showed significant cell morphological changes, accelerated aging of stem cells, and decreased differentiation ability compared to stem cells at passage 10 or less, such as passage 5. Thus, it was confirmed that culturing with a low passage number of passage 10 or less at a density of 1,000 cells/cm² or less is most effective.

### Example 2. Verification of improved subfractionation culturing method

Example 1 has confirmed that the control of cell density and passage and the addition of an antioxidant may be critical factors in the mesenchymal stem cell culture obtained by the subfractionation culturing method. Therefore, the experiments were conducted to compare the proliferation ability of single colony mesenchymal stem cells and their effect of obtaining cells, which were obtained by subculture during varying the cell culture density and varying a culture medium containing ascorbic acid as an antioxidant of monoclonal mesenchymal stem cells obtained by the conventional method, the subfractionation culturing method described in Korean Patent Application No. 10-2006-0075676.

Example 1 of the previous Korean Patent Application No. 10-2006-0075676 discloses a method for isolating and culturing mesenchymal stem cells from the bone marrow through the subfractionation culturing method as shown in FIG. 1, and discloses that the single cell population, colonies, obtained through the subfractionation culturing method is transferred to a culture vessel at the number of 100 to 600 cells per well.

In addition, Korean Patent Application No. 10-2014-0170045 discloses a method for isolating and culturing bone marrow-derived mesenchymal stem cells using a subfractionation culturing method and discloses that colonies are plated at 50 to 100 cells/cm².

However, Korean Patent Application Nos. 10-2006-0075676 and 10-2014-0170045 only disclose the conditions of colony culture corresponding to passage 1, the configuration to expand for the purpose of passage 1 to prepare seed cells for subculture in which the single cell population colonies obtained by the subfractionation culturing method were counted and transferred to a 6-well plate, and then plated at a low concentration. However, they do not disclose the configuration to the repeated culture cell density control of individual cells other than colonies after passage 2 and the effect thereof. According to the conventional subfractionation culturing method described in the above application, in order to obtain a sufficient amount of monoclonal stem cells having the effect of preventing, treating, and improving atopic dermatitis, the culture of at least 10 passages should be performed. On the other hand, the improved subfractionation culturing method of the present invention may effectively obtain a large amount of desired monoclonal stem cells through a low cell density condition after passage 2, and a small number of passages of up to 8 passages.

Specifically, in subculture after passage 2 (P2) after culturing the colonies of passage 1 (P1) obtained through the subfractionation culturing method in this improvement method, the cells were plated at a low density of 1,000 cells/cm² or less, and it was compared with the effect of 4,000 cells/cm² cell culture. In addition, the cell culture medium was varied depending on α-MEM medium containing antioxidants and LG-DMEM medium without antioxidants, and the cell proliferation effects were compared.

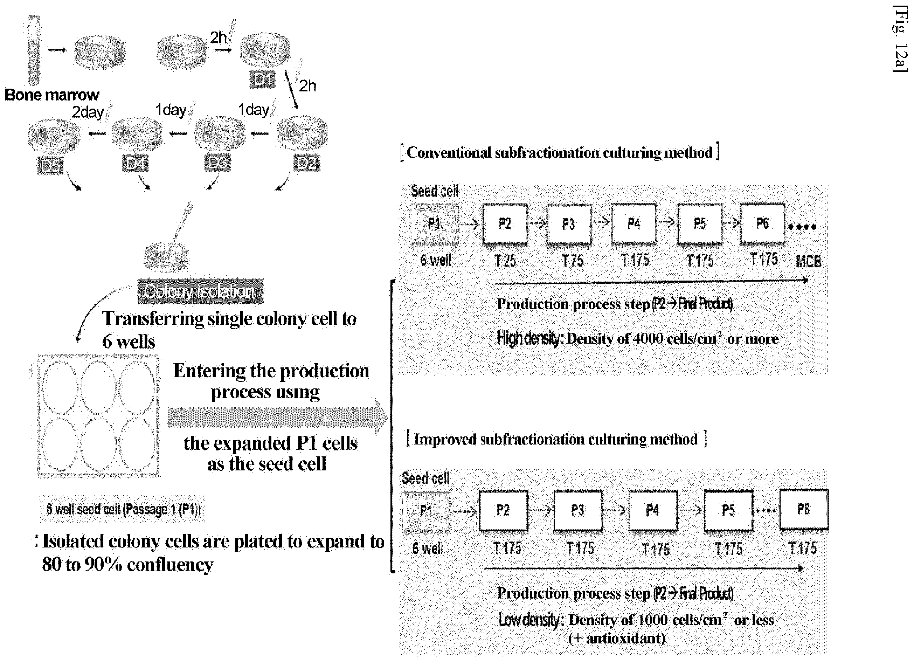

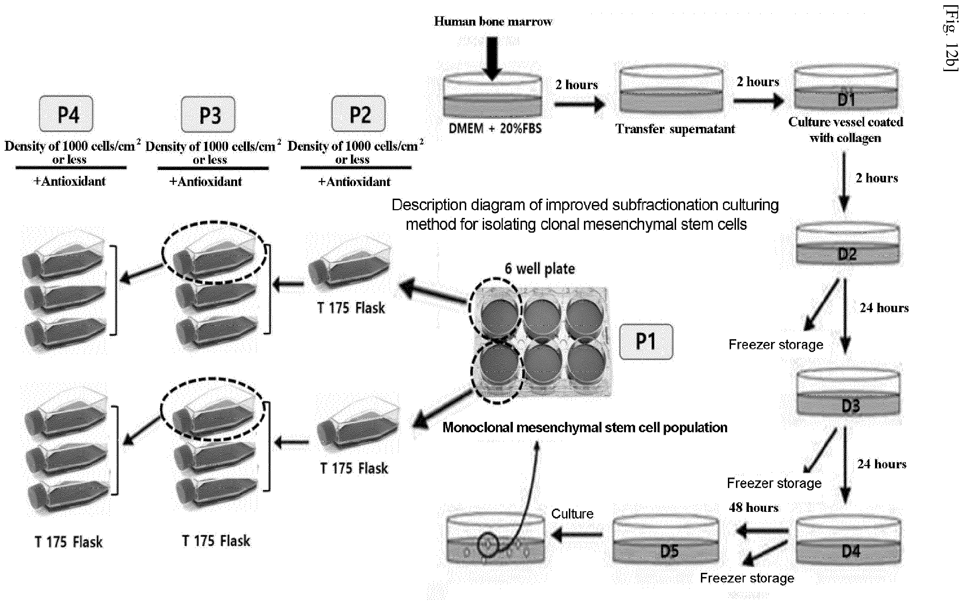

The experimental group for confirming the effect of the improved subfractionation culturing method is shown in Table 3 below, and the process improvement part of the improved subfractionation culturing method compared to the conventional subfractionation culturing method is schematically shown in FIGS. 12A and 12B.

As shown in FIG. 12A, the conventional subfractionation culturing method and the improved subfractionation culturing method proceed in the same manner as the process until the step of obtaining passage 1, but the improved subfractionation culturing method is different from the conventional subfractionation culturing method in the subculture process after the step of culturing by using the expanded passage 1 cells as seed cells. The conventional subfractionation culturing method performs high-density subculture of 4,000 cells/cm² or more for the purpose of obtaining many cells without recognition of density control, but the improved subfractionation culturing method controls the density of the subculture to a low density of 1,000 cells/cm² or less, thereby obtaining the final product only by culturing up to passage 8 after passage 2. The culture process after passage 2 is shown in detail in FIG. 12B.

**[Table 3]**

| **Group** | **Culture density** | **Medium conditions** | **Passage** | **Subculturing Period (Day)** | **Medium change** |
|---|---|---|---|---|---|
| 4000LG | 4000cells/cm² | LG-DMEM | | 3-4 | X |
| 4000alpha | 4000cells/cm² | alpha-MEM | | 3-4 | X |
| | | | P2-P5 | | |
| 1000LG | 1000cells/cm² | LG-DMEM | | 7 | O(every 3-4days) |
| 1000alpha | 1000cells/cm² | alpha-MEM | | 7 | O(every 3-4days) |

Cell lines in above Table 3 were separated by the subfractionation culturing method, which were named as SCM01 to SCM08, respectively.

### 2.1 Confirmation of proliferation effect according to cell line density and medium

The cells were cultured using the SCM01 to SCM08 cell lines. To confirm the cell proliferation effect according to subculture up to passage 5 (P5), which is less than passage 10, the cell number, cell population doubling time (PDT) and cell population doubling level (PDL) were compared, respectively. The results are illustrated in FIGS. 13 to 20.

As illustrated in FIGS. 13 to 20, the cell proliferation effect of all experimental groups inoculated and cultured with a cell density of 1,000 cells/cm² was superior to those of experimental groups inoculated and cultured with a cell density of 4,000 cells/cm². Furthermore, even in the same 1,000 cell density group, the 1,000-alpha experimental group cultured in α-MEM containing ascorbic acid as an antioxidant showed a more significant cell proliferation effect than other groups.

### 2.2 Comparison of proliferation effect according to cell line density

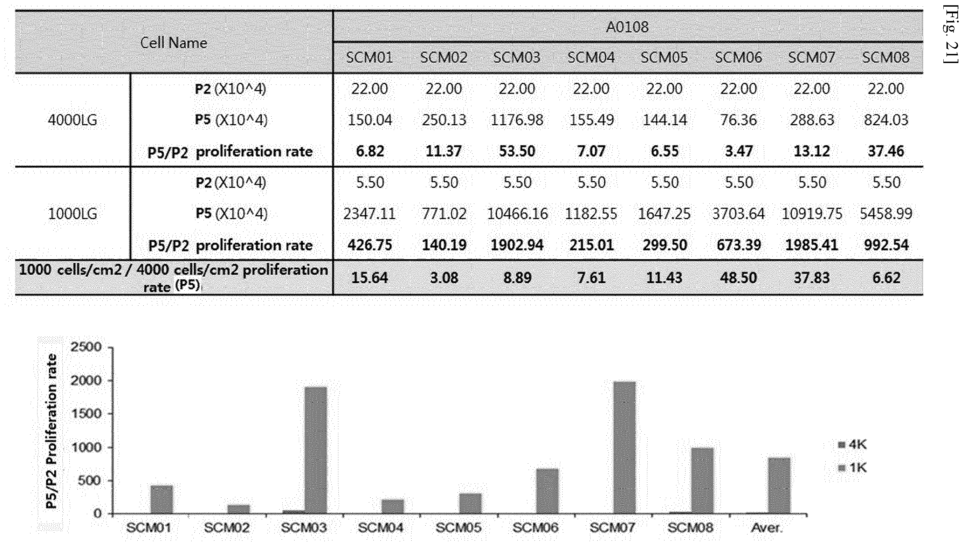

For a more accurate comparison of the proliferation rate according to the number of cultured cells, LG-DMEM and α-MEM, respectively, were set as a culture medium, and the cell inoculation density was set as 1,000 and 4,000 cells/cm², respectively. Accordingly, the cell proliferation effect according to subculture was compared. The results are illustrated in FIGS. 21 to 24.

As illustrated in FIG. 21, when the SCM01 to SCM08 cell lines inoculated with 1,000 cells/cm² were cultured in LG-DMEM, the proliferation rate of passage 2 (P2) to passage 5 (P5) was significantly higher than that of the group inoculated with 4,000 cells/cm². The proliferation rate of the group inoculated with 1,000 cells/cm² was at least 3.08 to at most 48.50 times compared with those of the group inoculated with 4,000 cells/cm² in passage 5 (P5).

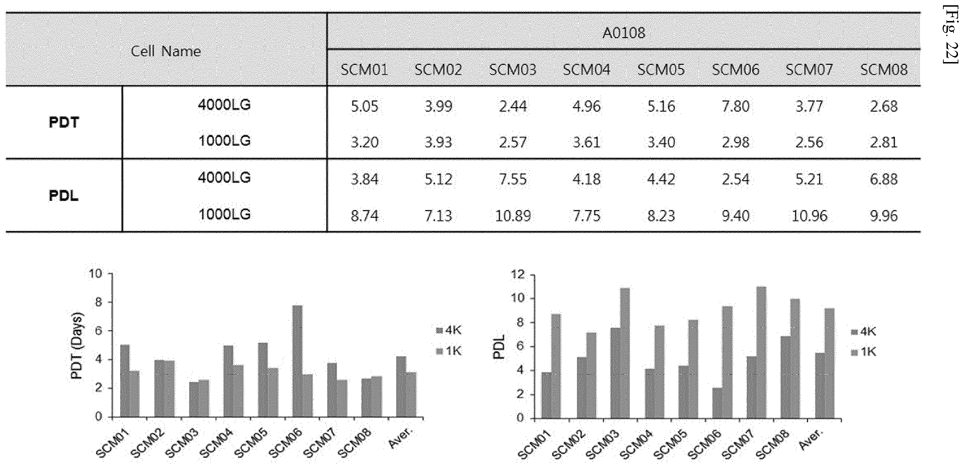

Further, as illustrated in FIG. 22, the PDT value of 1,000 cells/cm² inoculation group was also lower or similar to those of 4,000 cells/cm² inoculation in all cell lines, and the PDL value was higher than those of 4,000 cells/cm² inoculation in all cell lines.

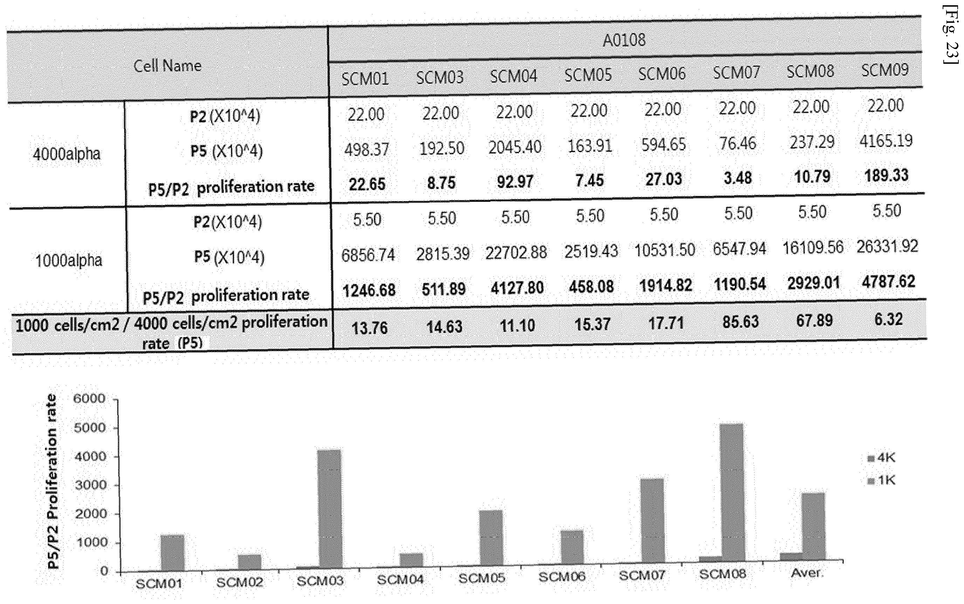

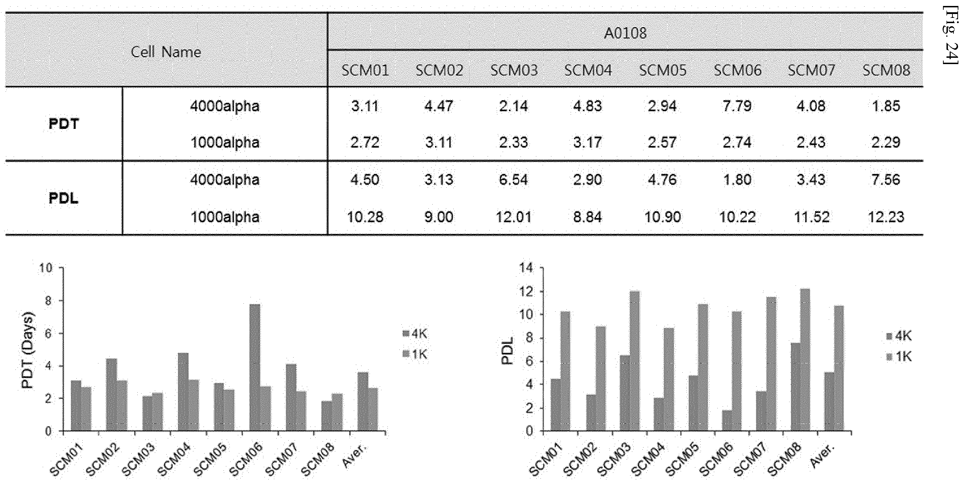

Further, as illustrated in FIG. 23, when all SCM01 to SCM08 cell lines cultured in α-MEM were inoculated and cultured with 1,000 cells/cm², their tendency was similar to those of DMEM experimental groups. The proliferation rate of the group inoculated with 1,000 cells/cm² was at least 6.32 to at most 85.63 times compared with those of the group inoculated with 4,000 cells/cm² in passage 5 (P5). Further, as illustrated in FIG. 24, the PDT value of 1,000 cells/cm² inoculation group was also lower or similar to those of 4,000 cells/cm² inoculation in all cell lines, and the PDL value was higher than those of 4,000 cells/cm² inoculation in all cell lines.

These results demonstrate that the cell inoculation with 1,000 cells/cm² or less may induce rapid proliferation of monoclonal mesenchymal stem cells compared to high density cell inoculation of 4,000 cells/cm².

### 2.3 Comparison of proliferation effect according to culture medium

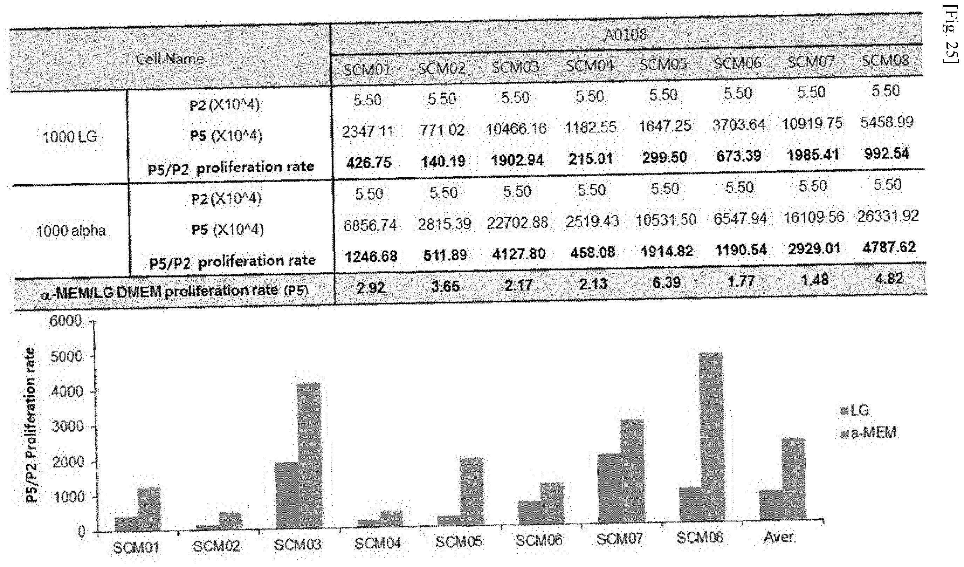

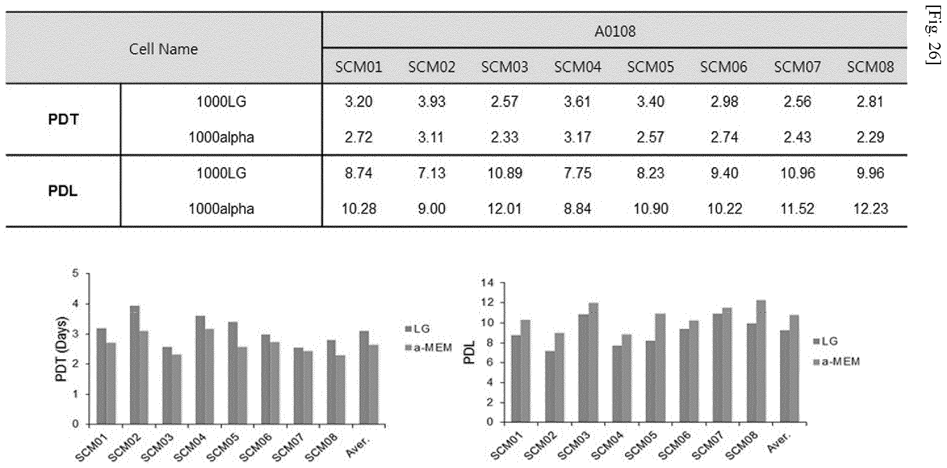

Example 2.2 confirmed that 1,000 cells/cm² culture showed excellent proliferation effect compared with 4,000 cells/cm² culture. Therefore, the experiment was conducted to compare the cell proliferation effect while the number of cells was set as 1,000 cells, and the medium varied as a variable. Thus, the proliferation effect was further verified according to the culture medium conditions. The results are illustrated in FIGS. 25 and 26.

As illustrated in FIG. 25, the cell proliferation rates were compared depending on α-MEM or DMEM as culture medium. The results showed that the proliferation rate of the group using α-MEM was at least 1.77 to 6.39 times higher compared with those of the group using LG-DMEM. Further, as illustrated in FIG. 26, the PDT was low in all α-MEM experimental groups and the PDL was increased in all α-MEM experimental groups.

These results indicate that the cell proliferation efficiency may be maximized by culturing cells using a medium containing an antioxidant in addition to manipulation of the cell inoculation density of 1,000 cells/cm² or less and culture of the cells in passage 2 (P2) to passage 5 (P5), which is low passage such as less than passage 10.

### Example 3. Establishment of improvement process

Examples, as described above, confirmed that the control of cell density and the addition of an antioxidant might be important factors in the mesenchymal stem cell culture. Thus, in addition to the conventional subfractionation culturing method described in Korean Patent Application Nos. 10-2006-0075676 and 10-2014-0170045, the improvedt process was established by varying the cell culture density and the medium conditions during subculture for effectively obtaining single colony mesenchymal stem cells in a low passage such as less than passage 10. These are collectively shown in the following Table 4 (culture conditions using DMEM) and Table 5 (culture conditions using α-MEM).

**[Table 4]**

| **Process** | **Items** | **Fresh Product** | **Frozen Product** |
|---|---|---|---|
| **Bone marrow ~MCB *seed cell (P1 exclusion)** | **Culture medium** | **DMEM** | **DMEM** |
| | **Antibiotic (concentration)** | **Penicillin-streptomycin** | **Penicillin-streptomycin** |
| | | **Penicillin (100units/mL)** | **Penicillin (100units/mL)** |
| | | **Streptomycin (100 ug/mL)** | **Streptomycin (100µg/mL)** |
| | **Cell culture density** | **50~1000cells/cm³** | **50~1000cells/cm²** |
| | **Subculturing Period** | **3 ~ 14 days** | **3 ~ 14 days** |
| | **Passage** | **P1~ P8** | **P1~ P8** |
| **MCB~WCB** | **Culture medium** | **DMEM** | **DMEM** |
| | **Antibiotic (concentration)** | **Penicillin-streptomycin** | **Penicillin-streptomycin** |
| | | **Penicillin (100units/mL)** | **Penicillin (100units/ml)** |
| | | **Streptomycin (100µg/mL)** | **Streptomycin (100µg/mL)** |
| | **Cell culture density** | **50~100cells/cm²** | **50~1000cells/cm²** |
| | **Subculturing Period** | **3 ~ 14 days** | **3 ~ 14 days** |
| | **Passage** | **P3~P5** | **P3~P5** |
| **Final product** | **Culture medium** | **DMEM** | **DMEM** |
| | **Antibiotic (concentration)** | **Penicillin-streptomycin** | **Penicillin-streptomycin** |
| | | **Penicillin (100units/mL)** | **Penicillin (100units/mL)** |
| | | **Streptomycin (100µg/mL)** | **Streptomycin (100µg/mL)** |
| | **Cell culture density** | **50~1000cells/cm²** | **50~1000cells/cm²** |
| | **Subculturing Period** | **3 ~ 14 days** | **3 ~ 14 days** |
| | **Passage** | **P6~P8** | **P6~P8** |

**[Table 5]**

| **Process** | **Items** | **Fresh Product** | **Frozen Product** |
|---|---|---|---|
| **Bone marrow ~MCB *seed cell (P1 exclusion)** | **Culture medium** | **α-MEM** | **α-MEM** |
| | **Antibiotic (concentration)** | **Gentamicin (20 µg/mL)** | **Gentamicin (20 µg/mL)** |
| | **Cell culture density** | **50~1000cells/cm²** | **50~1000cells/cm²** |
| | **Subculturing Period** | **3 ~ 14 days** | **3 ~ 14 days** |
| | **Passage** | **P1 ~ P8** | **P1~ P8** |
| **MCB~WCB** | **Culture medium** | **α-MEM** | **α~MEM** |
| | **Antibiotic (concentration)** | **Gentamicin (20 µg/mL)** | **Gentamicin (20µg/mL)** |
| | **Cell culture density** | **50~1000cells/cm²** | **50~1000cells/cm²** |
| | **Subculturing Period** | **3 ~ 14 days** | **3 ~ 14 days** |
| | **Passage** | **P3~P5** | **P3~P5** |
| **Final product** | **Culture medium** | **α-MEM** | **α-MEM** |
| | **Antibiotic (concentration)** | **Gentamicin (20 µg/mL)** | **Gentamicin (20 µg/mL)** |
| | **Cell culture density** | **50~1000cells/cm²** | **50~1000cells/cm²** |
| | **Subculturing Period** | **3 - 14 days** | **3 - 14 days** |
| | **Passage** | **P6~P8** | **P6~P8** |

More specifically, the subfractionation culture process and proliferation culture of the bone marrow-derived mesenchymal stem cells of the present disclosure were performed as follows.

After anesthetizing the hip of a bone marrow donor with local anesthetics, the bone marrow was collected by piercing the needle into the hip bone. 14 ml of Dulbecco's modified Eagle's medium (DMEM, GIBCO-BRL, Life-technologies, MD, USA) containing 20% FBS and 1% penicillin/streptomycin, and 1 ml of human bone marrow were placed in a 100 mm culture vessel, and the mixture was cultured in a 5% CO² cell incubator at 37°C for 2 hours. After the culture, the culture vessel was slightly tilted to one side, and only the supernatant of the culture vessel was transferred to a new vessel as much as possible while the cells attached to the bottom were prevented from falling down.

The same procedure was repeated one more time, and the resulting culture solution was transferred to a culture vessel (Becton Dickinson) coated with collagen on the bottom thereof and cultured at 37°C for 2 hours. The culture solution was transferred to a new vessel coated with collagen again. After 24 hours, the culture solution was transferred to the new vessel. Again, after 24 hours, the culture solution was transferred to a new vessel. Finally, after 48 hours, the culture solution was transferred to a new vessel. Then, it was visually confirmed that the remaining cells were grown and adhered to the bottom of the culture vessel. It can be assumed that cells that can come up to this step through the previous several subfractionation culture processes have a much smaller specific gravity than other cells.

After about 10 days to about 14 days, the cells formed a single colony. These monoclonal cell groups were treated with trypsin to be isolated. Then, the cells were transferred to a 6-well culture vessel. The cells were cultured in a 5% CO₂ cell incubator at 37°C for 4 to 5 days. Then, when the cells were grown to about 80%, the cells were treated with 0.05% trypsin/1 mM EDTA (GIBCO-BRL), thereby obtaining the cells. Then, the cells were transferred to T175 culture vessel and subcultured at a low cell density.

When the cells were cultured at a cell density which was reduced to 1000 cells/cm² in less than passage 10 as above described, preferably passage 8 or less such as the passage 2 (P2) to passage 5 (P5), although other procedures were regulated in the same manner, the proliferation ability and stem cell characteristics of mesenchymal stem cells were excellently maintained to induce efficient proliferation even in the same passage. In particular, when the cells are cultured at a cell density lowered, it may exclude a process of preparing a working cell bank (WCB) in mesenchymal stem cells, which is required in the conventional process, thereby shortening the cell production period efficiently. In particular, when the passage is reduced, cells with less aging may be obtained in a large amount. It is expected that such cells are used as a therapeutic agent to lead to excellent therapeutic effects.

Further, when α-MEM supplemented with an antioxidant is used as a culture medium, the antioxidant treatment may effectively reduce the ROS stress induced in high density cell culture and restore the cell proliferation ability of mesenchymal stem cells, thereby significantly shortening the cell passage and rapidly and stably obtaining single colony mesenchymal stem cells in a fresh state without aging, which maintains the characteristics of mesenchymal stem cells.

In summary, the low density cell culture may not only obtain a large number of cells in the short term, thereby simplifying the production process, but also obtain cells with non-aging to maintain the intact characteristics of mesenchymal stem cells in the long-term culture. Therefore, this leads to high quality stem cell production. Accordingly, stem cells obtained by the improved method constructed through the above Examples were used in a clinical study for the treatment of atopic dermatitis as below.

### Example 4. Establishment of atopic dermatitis treatment protocol of monoclonal mesenchymal stem cells

It is necessary to establish a clinical protocol that may induce atopic dermatitis therapeutic effect, which is applied to human atopic patients. Therefore, the allogeneic bone marrow-derived monoclonal stem cells obtained through the improved subfractionation culturing method of Examples 1 to 3 were applied to for emergency clinical trials of atopic patients as fresh (hereinafter, "SCM-CGH") and frozen (hereinafter, "SCM-AGH") types, and the therapeutic effect was confirmed.

### 4.1 Clinical participation patient conditions

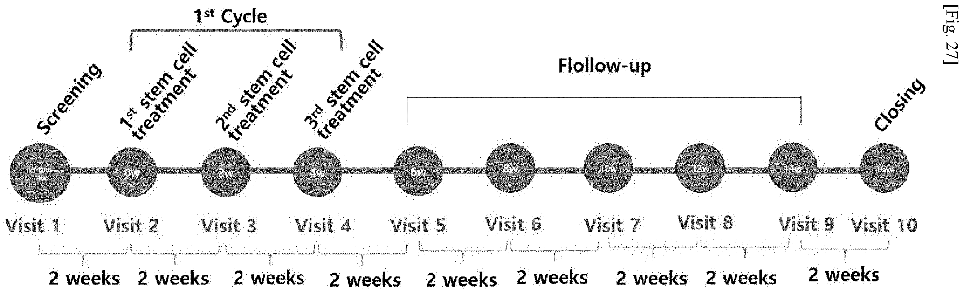

A schematic diagram of a clinical protocol for emergency atopic patients is shown in FIG. 27. Clinical trials were conducted at the College of medicine of Inha University (Incheon, Korea), and SCM-CGH or SCM-AGH was administered to patients for 24 months after clinical protocol approval. SCM-CGH or SCM-AGH was administered multiple times to adults aged 20 to 60 with the severe atopic disease to very severe atopic disease and the effects and safety were confirmed.

Atopic patients who participated in this clinical trial were targeted for patients with atopic symptoms for 6 months, and the clinical inclusion criteria and clinical exclusion criteria among atopic patients are shown in Table 6 below, and concomitant treatment and prohibited concomitant treatment criteria are shown in Table 7.

**[Table 6]**

| | |
|---|---|
| Inclusion criteria | - Men and women from 20 to 60 years of age |
| | - Patients diagnosed as atopic patients according to Hannifin and Rajka criteria |
| | - Patients with subacute or chronic atopic dermatitis lasting more than 6 months |
| | - Severe to very severe atopic patients with SCORAD score greater than 20 |
| | - Patient who signed informed consent form |
| Exclusion Criteria | - Patients with symptoms of systemic infection at first visit |
| | - Patients with asthma symptoms at first visit |
| | - Patients who received treatment with systemic steroids, systemic immunosuppressants, or systemic photochemotherapy within 4 weeks of visit |
| | - Patients who received topical steroids, oral antibiotics, or topical antibiotics within 2 weeks of visit |
| | - Patients who received treatment with drug for prohibited concomitant administration |
| | - Women of childbearing potential who are pregnant or breastfeeding, or who do not wish to use contraception during clinical trials |
| | - Patients who received other clinical trial drug treatment within 30 days prior to visit |
| | - Patients with creatinine > 2x upper limit of normal (ULN) at visit for screening |
| | - Patients with AST/ALT > 2x upper limit of normal (ULN) at visit for screening |
| | - Patients with hypersensitivity to antibiotics or antifungals |
| | - Patients who show serious side effects during stem cell therapy |
| | - Patients with medical or psychiatric risks that could affect study participation or analysis of study results in opinion of investigator |

**[Table 7]**

| | | |
|---|---|---|
| Concomitant treatment | Concomitant treatment of following drugs is permitted during clinical participation. | |
| | | 1. Concomitant treatment of drugs administered prior to participation in clinical trial, unless drug affects efficacy or stability of test drug |
| | | 2. Symptom treatment: emollients without corticosteroids, low-intensity (Group 6-7) topical corticosteroids, antihistamines, or antiinflammatory drugs |
| | | 3. Drugs temporarily used for treatment of other diseases or abnormal symptoms may be treated after consulting with clinician |
| | | 4. All detailed information related to concomitant treatment, including treatment of diseases other than atopic diseases, is recorded in CRF. |
| Prohibited Concomitant Treatment | Treatment of following drugs during clinical participation is prohibited | |
| | | 1. Immunomodulators |
| | | 2. High intensity (Group 1~5) topical steroids |
| | | 3. Systemic corticosteroids |
| | | 4. Systemic photochemical |
| | | 5. Live vaccine |
| | | 6. Other treatments that affect immune function |
| | If clinician determines that patients need concomitant treatment with a prohibited drug during clinical period, these patients are excluded from clinical trial, and this is recorded on last page of CFR. | |

### 4.2 Establishment of clinical protocols

The clinical trial was conducted in 3 visits for a total of 4 weeks, and additional cycles were determined according to the degree of the patient's symptoms. Specifically, SCM-CGH or SCM-AGH was administered at a concentration of 1 × 10⁶ cells/kg through the patient's vein. After the first administration, it was administered 2 times at 2-week intervals to be a total of 3 times (1st cycle). It was administered to the patient at a speed of 2 to 4.5 m/min for 10 minutes. In order to confirm the therapeutic effect on atopic dermatitis according to the administration, visual observation of the lesion and serum analysis of the patient were performed. From the 5th visit, after the 3rd administration of SCM-CGH or SCM-AGH, only serum was collected without SCM-CGH or SCM-AGH administration and used for analysis.

After the first cycle was completed, additional cycles were performed if the patient's symptoms recurred over time.

### 4.3 Method to check therapeutic effect on atopic dermatitis

The effect of SCM-CGH or SCM-AGH administration was confirmed by calculating, using EASI, SCORAD, BSA, VAS and IGA, the average value of the relative decrease in severity of atopic patients for 12 weeks after 3 administrations. Blood was drawn using SST (5 ml × 3 tubes at a total of 15 ml) at scheduled visits, and biomarker analysis was performed. Blood was collected before IP administration at the 2nd and 3rd IP administration visits (visit 3 and visit 4). A serum biomarker IgE; Th2 cytokines and chemokines IL4, IL13, CCL17 (TARC), CCL22 (MDC); a Th1 cytokine IL18; a Th17 cytokine IL17; and a Th22 cytokine IL22 were analyzed. Tolerability and safety were confirmed through side effects, mental reactions, vitals, blood and urine tests, and the occurrence of side effects was evaluated 4 hours after administration.

The contents of the evaluation performed during the clinical trial are shown in Table 8 below.

**[Table 8]**

| **Period** | **Screening** | **Treatment** | | | **Follow-up** | | | | | **Closing** |
|---|---|---|---|---|---|---|---|---|---|---|
| **Visit** | **1** | **2** | **3** | **4** | **5** | **6** | **7** | **8** | **9** | **10** |
| **Week** | **Within -4w** | **0w** | **2w±3d** | **4w±3d** | **6w±3d** | **8w±3d** | **10w±3d** | **12w±3d** | **14w±3d** | **16w±3d** |
| **Written consent** | ○ | | | | | | | | | |
| **Pre-treatment medications** | ○ | | | | | | | | | |
| **Demographic investigation** | ○ | | | | | | | | | |
| **Medical history** | ○ | | | | | | | | | |
| **Physical examination** | ○ | | | | | | | | | ○ |
| **Vital sign** | ○ | ○ | ○ | ○ | ○ | ○ | ○ | ○ | ○ | ○ |
| **Penicillin skin test** | ○ | | | | | | | | | |
| **Pregnancy test**¹⁾ | ○ | | | | | | | | | ○ |
| **Clinical laboratory test²⁾** | ○ | ○ | ○ | ○ | ○ | ○ | | ○ | | ○ |
| **Chest X-ray** | ○ | | | | | | | | | ○ |
| **ECG** | ○ | | | | | | | | | ○ |
| **Inclusion/Exclusion** | ○ | ○ | ○ | ○ | | | | | | |
| **SCORAD, IGA, EASI, BSA** | ○ | ○ | ○ | ○ | ○ | ○ | ○ | ○ | ○ | ○ |
| **Total IgE, ECP** | ○ | | ○ | ○ | ○ | | | | | ○ |
| **Biomarker** | ○ | | ○ | ○ | ○ | ○ | | ○ | | ○ |
| **Administration of Drug** | | ○ | ○ | ○ | | | | | | |
| **Adverse reactions** | | ○³⁾ | ○ | ○ | ○ | ○ | ○ | ○ | ○ | ○. |
| **Combination drugs** | | ○ | ○ | ○ | ○ | ○ | ○ | ○ | ○ | ○ |

| | | | | | | | | | | |
|---|---|---|---|---|---|---|---|---|---|---|
| **Hematology Hemoglobin, Hematocrit, RBC, WBC, WBC different cell count, Platelet** **Blood Chemistry Alkaline phosphatare, BUN, Creatinine, SGPT(ALT), SGOT(AST), Albumin, Total protein, Total bilirubin, Uric acid, Glucose, Cholesterol, Na, K, Cl, Calcium, Phosphorus** **Urinalysis: Specific Gravity, Color, pH, Protein, Glucose, Bilirubin, Blood, WBC** | | | | | | | | | | |

Eczema area and severity index (EASI) is used to measure the severity and degree of atopic dermatitis, and erythema, infiltration, excoriation and lichenification in four anatomical areas of the body (head, trunk, upper and lower extremities). The total EASI score ranges from 0 (minimum) to 72 (maximum), with high scores reflecting the severity of atopic dermatitis.

SCORing atopic dermatitis (SCORAD) is the most widely used internationally as a clinical tool to evaluate the severity of atopic dermatitis developed in Europe. The extent and intensity of eczema as well as subjective signs (such as insomnia) are assessed and scored. Factors such as the extent of the rash, the severity of the rash form, and subjective symptoms such as itchiness and sleep disturbance are evaluated in a 2 : 6 : 2 ratio. The total score ranges from 0 to 103 (severe).

The body parts of patients affected by atopic dermatitis were evaluated by body surface area (BSA). The highest score in each area was 9% for the head and neck, 18% for the anterior trunk, 18% for the back, 18% for the upper limbs, 36% for the lower limbs, and 1% for the genitals.

Visual analogue scale (VAS) is most commonly used in clinical trials for measuring the degree of itchiness and is characterized by high reliability and validity. The left endpoint represents "no itch" and the right endpoint represents "the most unimaginable itch." VAS 0, VAS <3, VAS ≥3 to <7, VAS ≥7 to <9, and VAS≥9, respectively, represent no itch, mild pruritus, moderate pruritus, severe itching and very severe itching.

Investigator's global assessment (IGA) is a method of evaluating the overall skin symptom level of atopic dermatitis in five stages. The score of 0, 1, 2, 3, and 4, respectively, represent transparent, almost transparent, weak normal, and severeness. An IGA score of 0 or 1 indicates a response to treatment.

### 4.4 Confirmation of atopic therapeutic effect

### 1) Patient information and administration procedures

Patients participating in the clinical trial and administration information are shown in Table 9 below.

**[Table 9]**

| **Subject No.** | **IP** | **1st Cycle** | | | **2nd Cycle** | | | | |
|---|---|---|---|---|---|---|---|---|---|
| | | **1^{st} Injection** | **2^{nd} Injection** | **3^{rd} Injection** | **1^{st} Injection** | **2^{nd} Injection** | **3^{rd} Injection** | **4^{th} Injection** | **5^{th} Injection** |
| **A (F/24)** | SCM-CGH | **2017-07-14** | | | | | | | |
| **B (M/21)** | SCM-CGH | **2017-11-22** | | | **2018-06-28** | | | | |

Patient A is a 24-year-old female, and according to the clinical protocol established in Example 4.2, SCM-CGH was additionally administered twice at an interval of 2 weeks after the first administration (1st cycle). The administration was performed a total of 3 times for 4 weeks, and serum was collected to confirm the therapeutic effect according to the evaluation method described in Example 4.3.

In patient B, SCM-CGH was additionally administered twice at an interval of 2 weeks after the first administration, for a total of 3 administrations for 4 weeks (1st cycle). 7 months after the end of the first cycle, the administration was performed 2 times at intervals of 2 weeks again. Thus, it was administered a total of 3 times for 4 weeks (2nd cycle).

### 2) Confirmation of patient's severity reduction

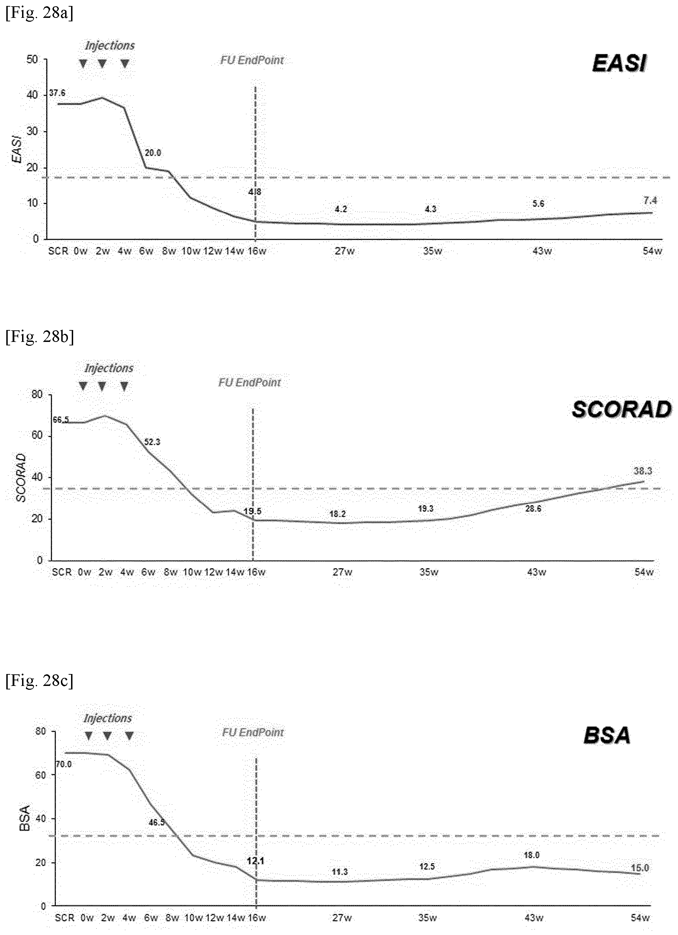

The effect of alleviating the severity of atopic dermatitis in patient A according to the administration of SCM-CGH is shown in FIG. 28.

As shown in FIG. 28A, patient A had a significant reduction in EASI, indicating the severity of atopic dermatitis after SCM-CGH administration. Specifically, in patient A, the EASI decreased by 87% at 12 weeks (i.e., 16 weeks after the start of the clinical trial) after the 1st cycle, and the EASI level was maintained to be 7.4 even at 54th week after 1 year or more after the 1st cycle, which was decreased by 80% or more.

As shown in FIG. 28B, SCORAD showed a tendency to gradually increase from the FU (flow-up) ending point to 54 weeks but showed a 70% reduction effect at the FU ending point.

As shown in FIG. 28C, BSA decreased at 12 weeks after the first cycle, and it appeared in a similar manner to EASI.

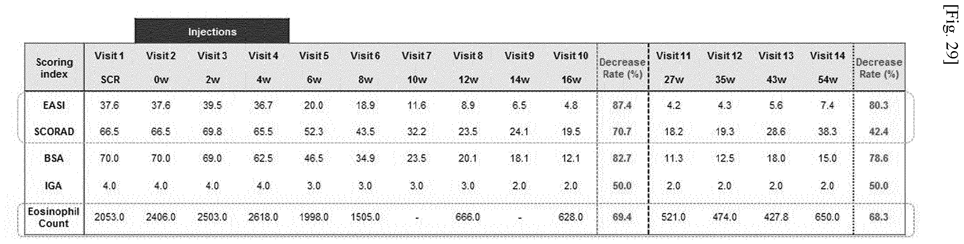

The results of EASI, SCORAD, BSA levels and IGA and eosinophil counts of patient A are shown in FIG. 29.

As shown in FIG. 29, a constant symptom ameliorating effect was confirmed in the atopic dermatitis evaluation index.

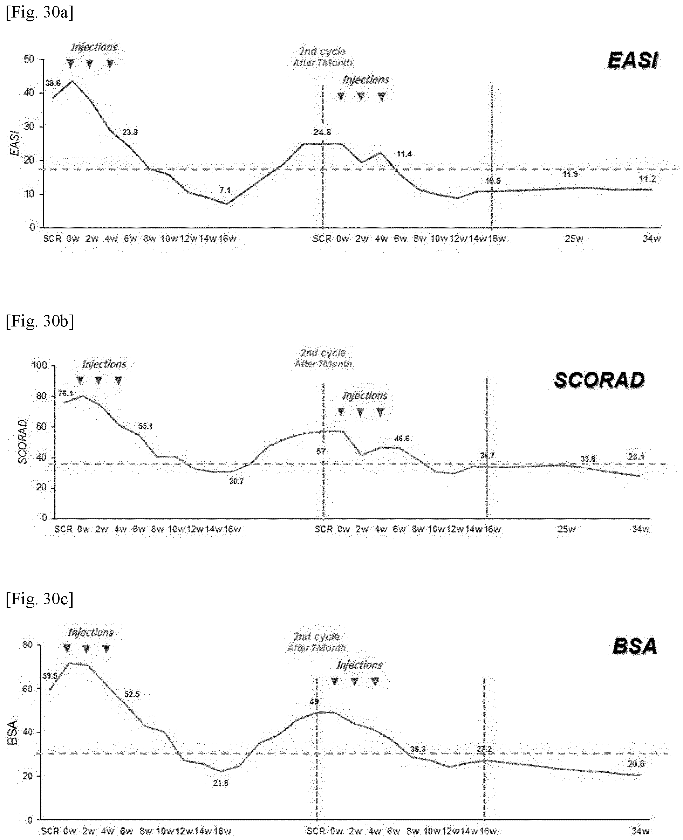

The effect of alleviating the severity of atopic dermatitis in patient B according to the administration of SCM-CGH is shown in FIG. 30.

Patient B showed the ameliorating of the atopic dermatitis symptoms after the first cycle, but it was found that the symptoms started again thereafter, and the second cycle was performed 7 months later.

As shown in FIG. 30A, in patient B, the EASI decreased by 80% or more at the 12th week after the first cycle (i.e., 16 weeks after the start of the clinical trial), and the EASI decreased by 50% or more after the second cycle.

As shown in FIG. 30B, SCORAD showed a tendency to decrease by 50% or more after the first cycle, and in particular, a continuous low level of SCORAD reduction was confirmed after the second cycle.

As shown in FIG. 30C, as a result of the BSA measurement, it was confirmed that patient B exhibited a very good BSA reduction tendency after the first cycle, and that the decreased level could be maintained constant for a long time after the second cycle.

In the case of patient B, the symptoms of atopic dermatitis alleviated very effectively only with the first cycle administration, but the symptoms recurred over time. When the same cycle was repeated, the levels decreased to a level smaller than the decrease in the first cycle. It was confirmed that these decreased levels may be maintained for a long time. Overall, in patient B, the EASI scoring decreased by 50% or more, and the EASI 72% and SCORAD 55% reduction levels were maintained about 60 weeks after the first administration. As a result of clinical application according to the present invention, it has been shown to have an alleviative effect on long-term atopic dermatitis.

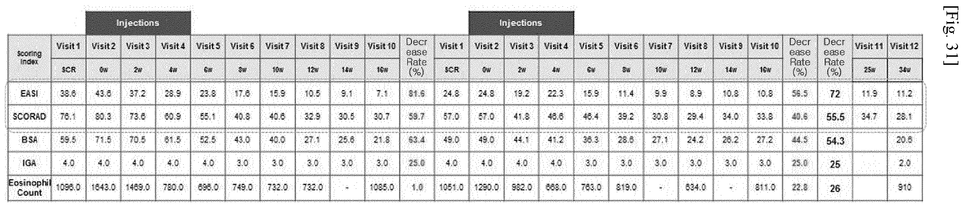

The results of EASI, SCORAD, BSA levels and IGA and eosinophil counts of patient B are shown in FIG. 31.

As shown in FIG. 31, it was confirmed that patient B also had the symptom alleviative effect evenly in the atopic dermatitis evaluation index by the administration of stem cells in the first and second cycles.

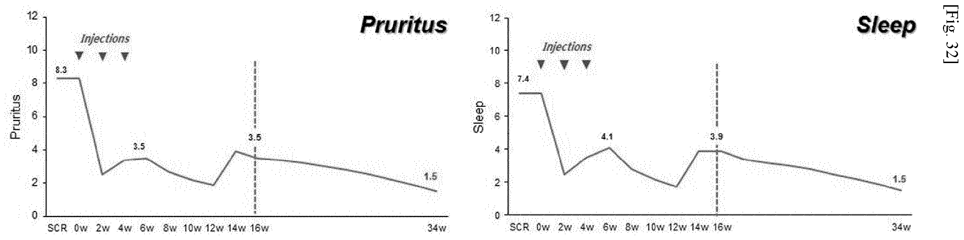

In addition, Pruritus and Sleep, which indicate itching and sleep disturbance scales, were investigated for patient B. For patient B, itching and sleep disturbance were rated on a score from 0 to 10, and the results are shown in FIG. 32.

As shown in FIG. 32, it was confirmed that itching and sleep disturbance in the patient were ameliorated by 50% or more after the second cycle, and the itching and sleep disturbance were very remarkably alleviated at the time point 34 weeks after the second cycle.

### 3) Confirmation of patient's serum analysis

In order to examine the diagnosis of allergic diseases and significant changes in severity from atopic dermatitis patients through molecular genetic analysis (ELISA), serum before and after injection of stem cell therapy was collected and analyzed. Specifically, sera from patients A and B were obtained and ELISA analysis was performed. The measurement may be stably performed in plasma, sputum, and bronchoalveolar fluid in addition to serum. CCL17 and CCL22, which can be used as biomarkers reflecting the diagnosis and severity of allergic diseases such as asthma and atopic dermatitis due to excellent reproducibility, were measured. The cytokines IL-18, IL-12 and IL-22 that promote the production of IFN-g from Th1 cells were measured.

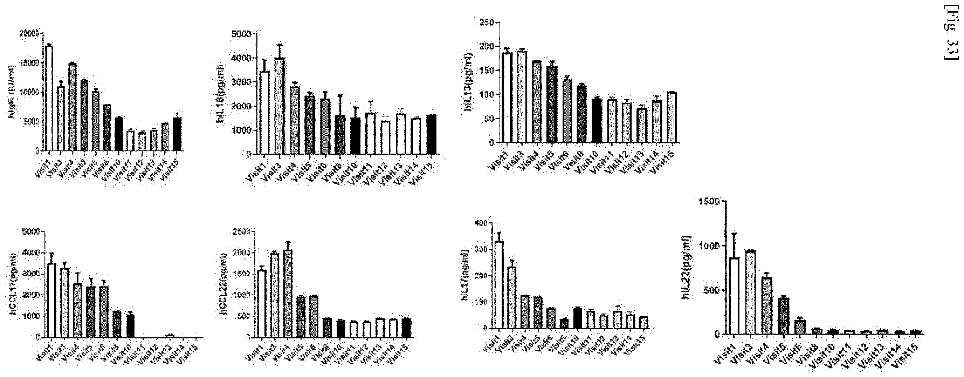

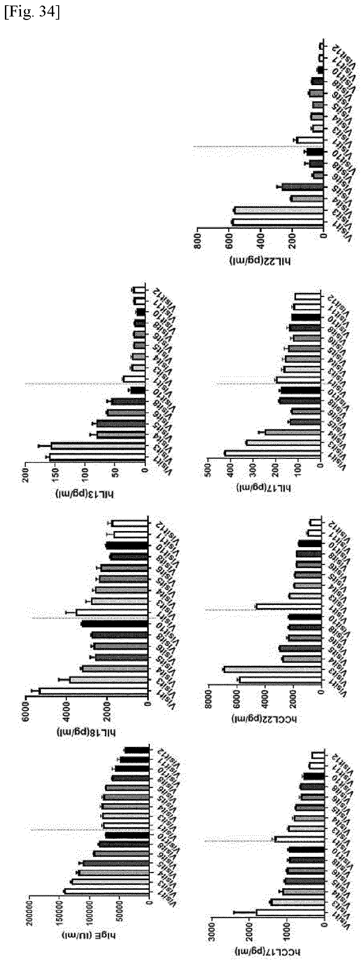

The results of measuring the effect in patient A and patient B are shown in FIGS. 33 and 34.

As shown in FIG. 33, it was confirmed that after administration of SCM-CGH to patient A, blood atopic-related factors continuously decreased according to the number of visits. Specifically, as a result of confirming the concentration of IgE, an atopic marker, it showed a tendency to decrease significantly. In addition, IL-18 and IL-13, which indicate the severity of atopic dermatitis, were also reduced, and IL-17, a marker that increases in dry lesions, was also significantly reduced. The blood levels of CCL17 and CCL22, which are biomarkers that reflect the diagnosis and severity of allergic diseases as Th2 chemokines, were also significantly reduced. In particular, it was confirmed that IL-22, which activates the innate immune response of the epidermis and increases in dry lesions, causing hyperproliferation of the epidermis, was significantly reduced.

As shown in FIG. 34, it was confirmed that patient B had similar results thereto as a result of analyzing changes in blood atopic-related factors after administration of SCM-CGH to patient B. The blood IgE concentration of patient B decreased after administration of the first cycle, and further decreased after administration of the second cycle. IL-18, IL-17 and IL-22 decreased and then increased after administration of the first cycle, but decreased after administration of the second cycle. The concentrations of IL-13, CCL17 and CCL22 in blood decreased after administration of the first cycle and further decreased after administration of the second cycle.

Through these results, it was confirmed that atopic dermatitis may be treated and alleviated in actual clinical practice when monoclonal mesenchymal stem cells according to the present invention are administered through the clinical protocol established in Example 4.2.

### 4.5 Selection of identification marker of atopic patient treatment group

According to the clinical protocol established in the present invention, Example 4.4 confirmed a very significant clinical atopic dermatitis treatment effect in patients A and B. However, although the alleviative effect on atopic dermatitis appeared in some patient groups, the clinical symptom alleviative effect was insignificant compared to the patients A and B groups. In particular, markers for screening atopic patient treatment groups suitable for the clinical protocol of the present invention were further analyzed. The patient group, which was confirmed that the treatment effect was relatively insignificant, was set as a control group of C to E, and information on the patient group is shown in Table 10.

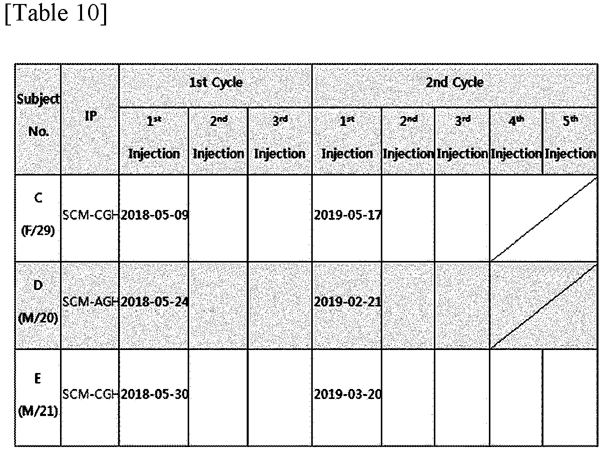

After obtaining blood before and after injection of a stem cell therapeutic agent to a patient, serum was separated from the blood and analyzed. Specifically, sera were obtained from patients A to E, and marker expression was compared and analyzed using an ELISA analysis program according to the manufacturer's protocol.

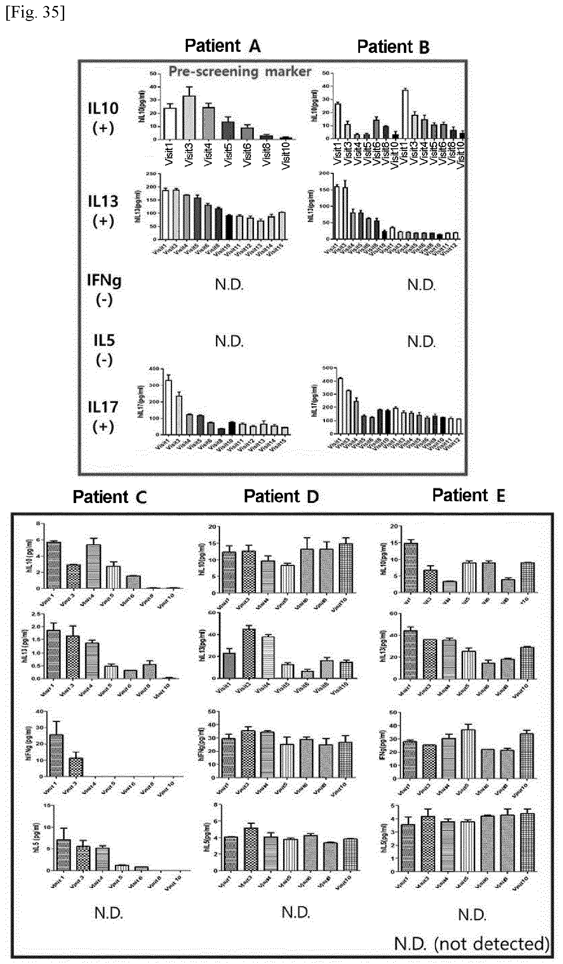

As a result of comparing the marker expression of the patient group A and B, which was confirmed to be suitable for application of the treatment method of the present invention because the therapeutic effect was very excellent, and the marker expression of the patient group showed a relatively low therapeutic effect, significant differences in expression were confirmed in IL-10, IL-13, IFNg and IL-5. The results are shown in FIG. 35.

As shown in FIG. 35, the A and B patient groups commonly showed high IL-10 and IL-13 expression patterns in which IL-10 is 20 pg/ml or more, and IL-13 is 100 pg/ml or more, compared to C to E patient groups showing a low expression in which IL-10 is 6 to 15 pg/ml, and IL-13 is 1.5 to 40 pg/ml.

In addition, surprisingly, it was confirmed that the expression of IFNg, IL-5 and IL-17 exhibited completely different patterns. In the case of patients A and B, the expression of IFNg and IL-5 was not detected before stem cell injection but the expression of IL-17 was detected. On the other hand, in the case of patients C to E, the expression of IFNg and IL-5 was detected, but the expression of IL- 17 was not detected.

This difference is a result showing that the prognosis may be predicted by pre-screening and confirming, through the measurement of the expression level of the marker, the atopic patient group particularly suitable for the treatment method of the present invention before application of the treatment method of the present invention.

## Claims

1. A composition for screening atopic dermatitis patients suitable for administration of monoclonal stem cells, the composition comprising one or more selected from the group consisting of IL-10, IL-13, IFNg, IL-5 and IL-17; or an agent for measuring their expression.

2. A composition for predicting prognosis of atopic dermatitis patients according to administration of monoclonal stem cells, the composition comprising one or more selected from the group consisting of IL-10, IL-13, IFNg, IL-5 and IL-17; or an agent for measuring their expression.

3. The composition of claim 1 or 2, wherein the monoclonal stem cells are obtained by steps of:
1) culturing bone marrow isolated from an individual in a first vessel;
2) transferring only a supernatant from the first vessel to a new vessel and culturing the supernatant
3) culturing the cells present in the new vessel and obtaining a supernatant;
4) repeating step 2) and 3) one or more times in which the supernatant of step 3) is used as the supernatant of the first vessel of step 2) to obtain the monoclonal stem cells; and
5) inoculating the monoclonal stem cells of step 4) in a medium at a cell density of 50 to 1,000 cells/cm² and culturing the cells to obtain the monoclonal stem cells.

4. A kit for screening atopic dermatitis patients suitable for administration of monoclonal stem cells, the kit comprising the composition of claim 1.

5. A kit for predicting prognosis of atopic dermatitis patients according to administration of monoclonal stem cells, the kit comprising the composition of claim 2.

6. A method for screening atopic dermatitis patients suitable for a method for administration of monoclonal stem cells, the method comprising a step of measuring expression of one or more selected from the group consisting of IL-10, IL-13, IFNg, IL-5 and IL-17 in a sample of atopic dermatitis patient.

7. The method of claim 6, wherein when the patient's
i) IL-10 is 20 pg/ml or more and IL-13 is 100 pg/ml or more,
ii) IFNg and IL-5 are not detected, or
iii) IL-17 is detected,
the patient is selected as a patient suitable for the method of the administration of the monoclonal stem cells.

8. A method for providing information on predicting prognosis of atopic dermatitis treatment according to administration of monoclonal stem cells, the method comprising a step of measuring expression of one or more selected from the group consisting of IL-10, IL-13, IFNg, IL-5 and IL-17 in a sample of atopic dermatitis patient.

9. The method of claim 8, wherein when the patient's
i) IL-10 is 20 pg/ml or more and IL-13 is 100 pg/ml or more,
ii) IFNg and IL-5 are not detected, or
iii) IL-17 is detected,
it is predicted that the prognosis is excellent on the administration of the monoclonal stem cells.

10. The method of any one of claims 6 to 9, wherein the monoclonal stem cells are obtained by steps of:
1) culturing bone marrow isolated from an individual in a first vessel;
2) transferring only a supernatant from the first vessel to a new vessel and culturing the supernatant;
3) culturing the cells present in the new vessel and obtaining a supernatant;
4) repeating step 2) and 3) one or more times in which the supernatant of step 3) is used as the supernatant of the first vessel of step 2) to obtain the monoclonal stem cells; and
5) inoculating the monoclonal stem cells of step 4) in a medium at a cell density of 50 to 1,000 cells/cm² and culturing the cells to obtain the monoclonal stem cells.

11. A pharmaceutical composition for preventing or treating atopic dermatitis comprising monoclonal stem cells, wherein the pharmaceutical composition is for administration
when before treatment, a patient's i) IL-10 is 20 pg/ml or more and IL-13 is 100 pg/ml or more, ii) IFNg and IL-5 are not detected, or iii) IL-17 is detected.

12. The pharmaceutical composition of claim 11, wherein the monoclonal stem cells are obtained by steps of:
1) culturing bone marrow isolated from an individual in a first vessel;
2) transferring only a supernatant from the first vessel to a new vessel and culturing the supernatant;
3) culturing the cells present in the new vessel and obtaining a supernatant;
4) repeating step 2) and 3) one or more times in which the supernatant of step 3) is used as the supernatant of the first vessel of step 2) to obtain the monoclonal stem cells; and
5) inoculating the monoclonal stem cells of step 4) in a medium at a cell density of 50 to 1,000 cells/cm² and culturing the cells to obtain the monoclonal stem cells.

13. A method of treating atopic dermatitis, the method comprising steps of:
1) culturing bone marrow isolated from an individual in a first vessel;
2) transferring only a supernatant from the first vessel to a new vessel and culturing the supernatant;
3) culturing the cells present in the new vessel and obtaining a supernatant;
4) repeating step 2) and 3) one or more times in which the supernatant of step 3) is used as the supernatant of the first vessel of step 2) to obtain the monoclonal stem cells;
5) inoculating the monoclonal stem cells of step 4) in a medium at a cell density of 50 to 1,000 cells/cm² and culturing the cells to obtain the monoclonal stem cells; and
6) administering the obtained monoclonal stem cells to the individual at a dose of 1 × 10³ to 1 × 10⁸ cells/kg at an interval of 1 week to 3 weeks.

14. The method of claim 13, wherein the monoclonal stem cells are a fresh type or frozen type.

15. The method of claim 13, wherein the medium in step 5) includes antioxidants.

16. The method of claim 13, wherein the culture in step 5) is cultured in passages 2 to 8.

17. The method of claim 13, wherein the administration of step 6) is performed 2 times to 5 times in one cycle.

18. The method of claim 17, wherein the monoclonal stem cells of step 5) are repeatedly administered in 1 cycle to 5 cycles.

19. The method of claim 13, wherein the administration is intravenous administration.

20. The method of claim 19, wherein the intravenous administration is performed at a rate of 0.5 m/min to 7 m/min for 5 minutes to 15 minutes.

21. The method of claim 13, wherein before treatment, IL-10 is expressed in 20 pg/ml or more, and IL-13 is expressed in 100 pg/ml or more in the individual.

22. The method of claim 13, wherein before treatment, IFNg and IL-5 are not expressed, and IL-17 is expressed in the individual.
